(19) Europäisches Patentamt
European Patent Office
Office européen des brevets

(11) Veröffentlichungsnummer: **0 004 358**
**B1**

# (12) EUROPÄISCHE PATENTSCHRIFT

(45) Veröffentlichungstag der Patentschrift: **06.01.82**

(51) Int. Cl.³: **C 07 D 405/14**, C 07 D 411/14, A 61 K 31/495, A 61 K 31/445

(21) Anmeldenummer: **79100815.4**

(22) Anmeldetag: **16.03.79**

(54) **N-Oxacyclyl-alkyl-piperidine, Verfahren zu ihrer Herstellung, pharmazeutische Präparate enthaltend diese Verbindungen.**

(30) Priorität: **20.03.78 US 888089**

(43) Veröffentlichungstag der Anmeldung: **03.10.79 Patentblatt 79/20**

(45) Bekanntmachung des Hinweises auf die Patenterteilung: **06.01.82 Patentblatt 82/1**

(84) Benannte Vertragsstaaten: **BE CH DE FR IT LU NL SE**

(56) Entgegenhaltungen: **DE-A-2 400 094**

(73) Patentinhaber: **CIBA-GEIGY AG, Patentabteilung Postfach, CH-4002 Basel (CH)**

(72) Erfinder: **Huebner, Charles Ferdinand, Dr., 40 Edgewood Road, Chatham New Jersey (US)**

(74) Vertreter: **Zumstein sen., Fritz, Dr. et al, Bräuhausstrasse 4, D-8000 München 2 (DE)**

EP 0 004 358 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

N-Oxacyclyl-alkyl-piperidine, Verfahren zu ihrer Herstellung, pharmazeutische Präparate enthaltend diese Verbindungen

In der DE 2 400 094 sind 1-[1-[2-(1,4-Benzodioxan-2-yl)-2-hydroxyäthyl]-4-piperidyl]-2-benzimidazolionone, welche eine ausgeprägte blutdrucksenkende Wirkung haben, beschrieben, Es ist nun überraschenderweise gefunden worden, daß die N-Oxacyclyl-alkyl-piperidine, nämlich die 1,4-Benzodioxan-2-yl-niederalkyl-4-piperidyl-Verbindungen und die entsprechenden Benzoxathiandefivate, welche anstelle der Hydroxyäthylgruppe einen unsubstituierten Niederalkylrest und anstelle des Benzimidazolinonrestes eine Gruppe der Formel

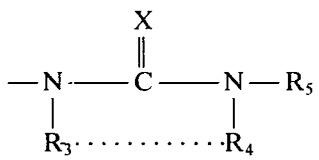

besitzen, worin die einzelnen Symbole die für die Formel I angegebenen Bedeutungen haben, stimulierende und antidepressive Effekte aufweisen.

Die Erfindung betrifft diese neuen N-Oxacyclyl-alkyl-piperidyl-diazaverbindungen der allgemeinen Formel I

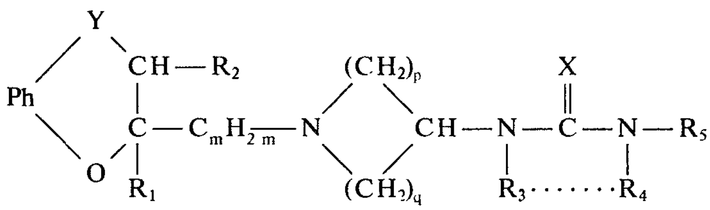

(I)

worin Ph unsubstituiertes 1,2-Phenylen oder 1,2-Phenylen, welches durch einen bis drei gleiche oder verschiedene Substituenten der Gruppe bestehend aus Niederalkyl, Niederalkoxy, Niederalkylendioxy, Halogen oder Trifluormethyl substituiert ist, bedeutet, jedes der Symbole $R_1$ und $R_2$ für Wasserstoff oder Niederalkyl steht, jedes der Symbole $R_3$ und $R_4$ Wasserstoff oder Niederalkyl bedeutet oder $(R_3+R_4)$ für Ph oder Niederalkylen steht, welches die beiden Stickstoffatome durch 2 bis 4 Kohlenstoffatome trennt, $R_5$ Wasserstoff, Niederalkyl oder HPh bedeutet, X für Oxo, Thioxo, Imino oder Niederalkylimino steht, Y Epoxy, Epithio oder Sulfinyl bedeutet, m für eine ganze Zahl von 1 bis 7 steht, jedes der Symbole p und q eine ganze Zahl von 1 bis 3 bedeutet, wobei $(p+q)$ die Zahl 4 ist, sowie Säureadditionssalzen, insbesondere therapeutisch verwendbaren Säureadditionssalzen dieser Verbindungen.

Ein 1,2-Phenylenrest Ph ist vorzugsweise unsubstituiert oder monosubstituiert, und seine höchstens drei Substituenten werden durch die folgenden Gruppen illustriert: Niederalkyl, z. B. Methyl, Äthyl, n- oder i-Propyl oder -Butyl; Niederalkoxy, z. B. Methoxy, Äthoxy, n- oder i-Propoxy oder -Butoxy, Niederalkylendioxy, z. B. Methylendioxy, 1,1- oder 1,2-Äthylendioxy, Halogen, z. B. Fluor, Chlor oder Brom oder Trifluormethyl.

Jedes der Symbole $R_1$ bis $R_5$ bedeutet vorzugsweise Wasserstoff, aber auch Niederalkyl, insbesondere Methyl, oder einen anderen obengenannten Niederalkylrest. Das Symbol $R_5$ kann auch für Phenyl stehen, welches unsubstituiert oder substituiert sein kann, wobei die Substituenten diejenigen eines H-Ph-Restes sind. Die Symbole $R_3$ und $R_4$ können gemeinsam auch den Rest Ph darstellen oder für Niederalkylen stehen, welches die zwei Stickstoffatome insbesondere durch 2 oder 3 Kohlenstoffatome trennt, z. B. für 1,2-Äthylen, 1,2- oder 1,3-Propylen, 1,2-, 1,3- oder 2,3-Butylen. Das Symbol X ist vorzugsweise Oxo, aber auch Thioxo, Imino oder Niederalkylimino und Y bedeutet vorzugsweise Epoxy, aber auch Epithio oder Sulfinyl. Von den genannten ganzen Zahlen ist m vorzugsweise 1 bis 4 und $C_mH_{2m}$ bedeutet vorzugsweise Methylen, 1,1- oder 1,2-Äthylen, 1,2- oder 1,3-Propylen, 1,2-, 1,3- oder 1,4-Butylen, und jedes der Symbole p und q steht vorzugsweise für 2.

Alle basischen Verbindungen der allgemeinen Formel I können in Form ihrer Säureadditionssalze, insbesondere therapeutisch verwendbare Säureadditionssalze, welche z. B. von untengenannten Säuren abgeleitet sind, vorliegen.

Der Ausdruck »nieder« definiert in den oben oder nachfolgend genannten organischen Resten oder Verbindungen solche mit höchstens 7, vorzugsweise 4, insbesondere 1 oder 2 Kohlenstoffatomen.

Die Verbindungen der Erfindung zeigen wertvolle pharmakologische, z. B. stimulierende und antidepressive Wirkungen. Diese können in Tierversuchen, vorzugsweise an Säugetieren, wie Ratten oder Affen, als Testobjekte nachgewiesen werden. Die Verbindungen der Erfindung können ihnen enteral, z. B. oral, oder parenteral, z. B. subkutan, intraperitoneal oder intravenös, z. B. in Form von

wässerigen Lösungen oder Stärke enthaltenden Suspensionen, verabreicht werden. Die verwendete Dosis kann in einem Bereich von ungefähr zwischen 0,1 und 100 mg/kg/Tag, vorzugsweise ungefähr 1 und 50 mg/kg/Tag, insbesondere ungefähr 5 und 25 mg/kg/Tag liegen. Die genannten stimulierenden und antidepressiven Wirkungen können z. B. an männlichen Albinoratten beobachtet werden. Die Tiere haben freien Zugang zu Futter und Wasser, mit Ausnahme der Versuchsdauer. Die Untersuchung des Tierverhaltens wird in normierten, schallisolierten Konditionierungskammern, welche einen Schalthebel enthalten, durchgeführt. Gemäß dem Test werden die Ratten durch das Bodengitter der Kammer elektrischen Schocks ausgesetzt. Der Hebel ist an eine programmierte Einrichtung, welche die Abgabe von elektrischen Schocks reguliert, angeschlossen. Die Anzahl der Betätigung des Hebels und die Anzahl der erlittenen Schocks werden registriert. Die Ratten werden vorher dazu trainiert, diesem elektrischen Impuls durch Drücken der Taste auszuweichen. Dann wird der Apparat so eingestellt, daß jede Hebelbetätigung den Schockeintritt um 30 Sekunden verschiebt. Vergißt das Tier, den Hebel innerhalb dieses Zeitintervalls zu drücken, so werden alle 15 Sekunden kurze elektrische Schocks abgegeben, bis das Tier den Hebel wieder betätigt. Vor jeder Testperiode werden die Ratten, um sich zu erwärmen, 15 Minuten in den Tastkammern gehalten. Während dieser Zeit wird die Anzahl der Hebelbetätigungen nicht registriert. Unmittelbar nach dieser Periode werden die Test-Verbindungen entweder in Kochsalzlösung oder in 3%iger Maisstärke-Suspension in 5%igem wässerigem Polyäthylenglykol-400, welches auf 10 ml einen Tropfen Polyäthylen-20-sorbitanmonooleat enthält, entweder oral oder intraperitoneal verabreicht. So erhöht signifikant z. B. das 1-[1-[2-(1,4-Benzodioxan-2-yl)-äthyl]-4-piperidyl]-2-imidazolidinon, insbesondere seine linksdrehende Form, z. B. das 1-(S)-Hydrobromid oder -Fumarat — typische Vertreter von Verbindungen der Formel I —, die Schockvermeidungsreaktion der Ratte. Der Wirkstoff wird intraperitonal in Dosen bis hinunter zu 2,5 mg/kg/Tag verabreicht. Die ermittelte Zunahme der Schockvermeidungsreaktion läßt sich vorteilhaft mit derjenigen, die eine Dosis von 5 mg/kg/Tag Methylphenidat (ein klassisches Stimulans) bei intraperitonealer Verabreichung hervorruft, vergleichen. Diese erfindungsgemäße Verbindung zeigt einen einzigartigen Wirkungsmechanismus. Sie ist offenbar kein Aufnahmehemmer biogener Amine, kein präsynaptischer $\alpha$-noradrenergischer Blocker wie das Mianserin und kein Amphetamin ähnliches, suchterregendes Stimulans. Sie besitzt ferner weder eine anticholinergische noch eine antihistaminische Wirkung, d. h., die Verbindung ist frei von diesen üblichen, bei bekannten Antidepressiva auftretenden Nebenwirkungen.

In einem anderen Test werden Totenkopf-Äffchen (squirrel monkeys) durch Training dazu gebracht, den Hebel in einem solchen Skinner-Käfig zu drücken, um dem elektrischen Schock, der auf die Füße durch das Bodengitter verabreicht wird, auszuweichen. Jeder Tastendruck verschiebt den elektrischen Schock um 20 Sekunden. Versäumt der Affe, innerhalb von 20 Sekunden die Taste zu drücken, so wird er kurze (0,5 Sekunden) elektrische Schocks alle 20 Sekunden bis zu seinem nächsten Tastendruck erleiden. Unter Kontrollbedingungen drücken die Affen den Hebel mit einer verhältnismäßig stetigen Geschwindigkeit, so daß sie selten mehr als 6 Schocks während einer vierstündigen Versuchsperiode erhalten. Man bestimmt die Anzahl der Vermeidungsreaktionen sowie die Anzahl der erhaltenen Schocks. Bei oraler Verabreichung in 0,9%iger wässeriger Kochsalzlösung ruft das genannte Hydrobromid oder Fumarat positive Änderungen sowohl im Ausmaß der Vermeidungsreaktionen als auch in der Anzahl der erlittenen Schocks hervor. Der Wirkstoff wird in niedrigen Dosen bis hinunter zu 2,5 mg/kg/Tag verabreicht. Die Untersuchung zwecks Gewinnung von Kontrollwerten wird unter Verwendung von Kochsalzlösung allein, ein Tag vor der Untersuchung der Wirkstoffe, durchgeführt.

Die Verbindungen der Erfindung sind dementsprechend wertvolle Psychostimulantia, z. B. in der Behandlung oder Handhabung von Depressionen oder geringen Gehirnfunktionsstörungen. Die neuen Verbindungen können überdies als Zwischenprodukte zur Herstellung von anderen wertvollen, insbesondere von pharmakologisch wirksamen Verbindungen, eingesetzt werden.

Bevorzugte Verbindungen sind diejenigen der allgemeinen Formel I, worin Ph unsubstituiertes oder durch Niederalkyl, Niederalkoxy, Niederalkylendioxy, Halogen oder Trifluormethyl monosubstituiertes 1,2-Phenylen bedeutet, jedes der Symbole $R_1$ und $R_2$ für Wasserstoff oder Niederalkyl steht, jedes der Symbole $R_3$ und $R_4$ Wasserstoff oder Niederalkyl ist oder $(R_3+R_4)$ den Rest Ph oder Niederalkylen bedeutet, welches die beiden Stickstoffatome durch 2 oder 3 Kohlenstoffatome trennt, das Symbol $R_5$ für Wasserstoff, Niederalkyl oder HPh steht, X Oxo, Thioxo, Imino oder Niederalkylimino bedeutet, Y für Epoxy, Epithio oder Sulfinyl steht, m für eine ganze Zahl von 1 bis 4 steht, jedes der Symbole p und q eine ganze Zahl von 1 bis 3 bedeutet, wobei $(p+q)$ die Zahl 4 ist, und therapeutisch verwendbare Säureadditionssalze dieser Verbindungen.

Bevorzugt sind weiter Verbindungen der allgemeinen Formel I, worin Ph unsubstituiertes oder durch Alkyl oder Alkoxy, jeweils mit höchstens 4 Kohlenstoffatomen, Halogen oder Trifluormethyl monosubstituiertes 1,2-Phenylen bedeutet, jedes der Symbole $R_1$, $R_2$ und $R_5$ für Wasserstoff oder Alkyl mit höchstens 4 Kohlenstoffatomen steht, jedes der Symbole $R_3$ und $R_4$ Wasserstoff bedeutet oder $(R_3+R_4)$ für Alkylen mit 2 bis 4 Kohlenstoffatomen steht, welches die beiden Stickstoffatome durch 2 oder 3 Kohlenstoffatome trennt, X Oxo, Thioxo oder Imino bedeutet, Y für Epoxy oder Epithio steht, m für eine ganze Zahl von 1 bis 4 steht, jedes der Symbole p und q 2 bedeutet, und therapeutisch verwendbare Säureadditionssalze dieser Verbindungen.

Besonders hervorzuheben sind Verbindungen der allgemeinen Formel II

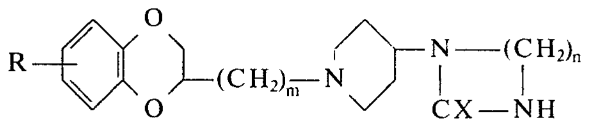

(II)

worin R Wasserstoff, Alkyl oder Alkoxy, jeweils mit höchstens 4 Kohlenstoffatomen, Halogen oder Trifluormethyl bedeutet, m für eine ganze Zahl von 1 bis 4 steht, n die ganze Zahl 2 oder 3 bedeutet, und X ist Oxo, Thioxo oder Imino, und therapeutisch verwendbare Säureadditionssalze dieser Verbindungen.

Die Verbindungen der vorliegenden Erfindung werden nach an sich bekannten Methoden, z. B. dadurch hergestellt, daß man

a) einen reaktionsfähigen Ester eines oxacyclischen Alkanols der allgemeinen Formel III mit einer 1-unsubstituierten Piperidyl-diaza-Verbindung der allgemeinen Formel IV kondensiert:

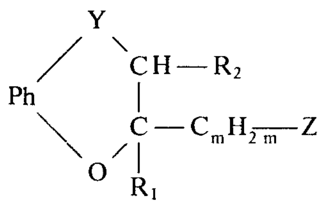

+

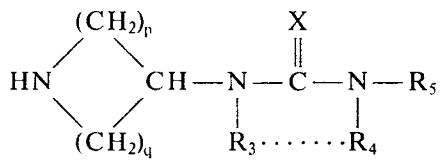

(III) (IV)

worin Z eine reaktionsfähige veresterte Hydroxygruppe bedeutet.

Eine reaktionsfähige veresterte Hydroxygruppe Z ist z. B. eine mit einer starken anorganischen oder organischen Säure, vor allem einer Halogenwasserstoffsäure, z. B. Chlorwasserstoffsäure, Bromwasserstoffsäure oder Jodwasserstoffsäure, Schwefelsäure oder einer aromatischen Sulfonsäure, z. B. p-Toluolsulfonsäure oder m-Brombenzolsulfonsäure veresterte Hydroxygruppe. Die Kondensation wird vorzugsweise in Gegenwart von basischen Kondensationsmitteln, z. B. Alkalimetall- oder Erdalkalimetall-hydroxyden, -carbonaten oder -hydrogencarbonaten, wie Natrium, Kalium- oder Calcium-hydroxyd oder -carbonat, Alkalimetallhydriden, -niederalkoxiden oder -niederalkanoaten, wie Natriumhydrid, Natriummethylat oder Natriumacetat, oder von organischen tertiären Stickstoffbasen, wie Triniederalkylaminen oder Pyridinen, z. B. Triäthylamin oder Lutidin, durchgeführt.

Ein weiteres Verfahren zur Herstellung der neuen Verbindungen besteht darin, daß man

b) eine Verbindung der allgemeinen Formel V mit dem Kohlensäurederivat der allgemeinen Formel VI umsetzt:

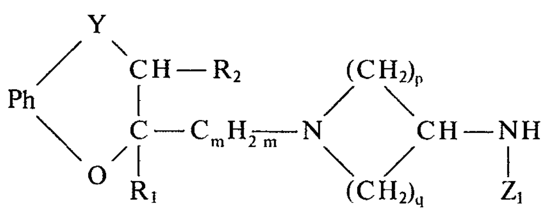

+ $Z_2CX'$

(V) (VI)

worin $Z_1$ für $R_3$ steht oder die Gruppierung $R_3 \ldots R_4-NH-R_5$ bedeutet und $Z_2CX'$ für Ammonium-cyanat oder -thiocyanat, ein Metall-cyanat oder -thiocyanat, einen Niederalkyl-isoharnstoff oder -isothioharnstoff, ein Halogencyan oder Cyanamid, Schwefelkohlenstoff oder Carbonylsulfid, ein Kohlensäurehalogenid oder 1,1-Carbonyldiimidazol bedeutet, mit der Maßgabe, daß mindestens eine der Gruppen $Z_1$ und $Z_2$ Stickstoff enthält.

In dieser Reaktion wird entweder eine Carbamoylgruppe an die Verbindung V addiert (wenn $Z_1$ für $R_3$ steht) oder die Gruppe CX in die Verbindung V eingebaut (wenn $Z_1$ die Gruppierung der Formel $R_3 \ldots R_4-NH-R_5$ bedeutet). Das genannte Cyanat ist vorzugsweise ein Alkalimetallcyanat, ein Halogencyan, insbesondere das Bromcyan, und ein Kohlensäurehalogenid, vorzugsweise das Phosgen. Das Verfahren wird in an sich bekannter Weise, je nach der Bedeutung von $Z_2$, durchgeführt. Ist $Z_2$ metallisch, so wird die Reaktion in einem neutralen oder sauren Lösungs- oder Verdünnungsmittel, z. B. in einem mit Wasser mischbaren polaren Lösungsmittel, wie in einem

wässerigen Niederalkanol, -alkanon, oder in einem gesättigten cyclischen Äther, z. B. Äthanol, Aceton, Tetrahydrofuran oder Dioxan, oder in einem alkylierten Formamid oder Sulfoxyd, z. B. Dimethylformamid oder Dimethylsulfoxyd, durchgeführt. Enthält die Gruppe $Z_2$ kein Metall, so kann ein basisches Mittel für die Bindung der entstandenen Säure verwendet werden. Solche Mittel sind ein Alkalimetall- oder Erdalkalimetall-hydroxyd, -carbonat oder -hydrogencarbonat, z. B. Natrium-, Kalium- oder Calcium-hydroxyd oder -carbonat, Alkalimetallhydride, -niederalkoxide oder -alkanoate, wie Natriumhydrid, -methylat oder -acetat, aber auch organische tertiäre Stickstoffbasen, wie Tri-niederalkylamine oder Pyridine, z. B. Triäthylamin oder Lutidin.

Ein weiteres Verfahren zur Herstellung der neuen Verbindungen besteht darin, daß man

c) eine Verbindung der allgemeinen Formel VII

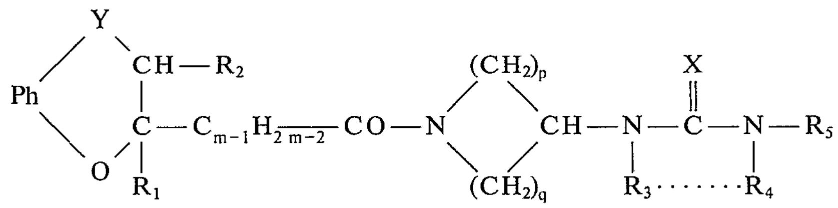

(VII)

reduziert.

Die Reduktion wird in an sich bekannter Weise, vorzugsweise mit einfachen oder komplexen Leichtmetallhydriden, wie Diboran oder Alan, Alkalimetall-borhydriden, Alkalimetallaluminiumhydriden oder Alkalimetall-alkoxyborhydriden, z. B. Lithiumaluminiumhydrid und/oder Natriumtrimethoxyborhydrid, durchgeführt.

Ein weiteres Verfahren zur Herstellung der neuen Verbindungen besteht darin, daß man

d) eine Verbindung der allgemeinen Formel VIII

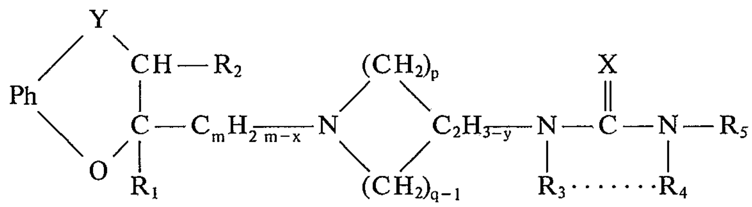

(VIII)

worin jedes der Symbole x und y die Zahl 0 oder 2 bedeutet und ihre Summe x+y für 2 oder 4 steht, hydriert.

Die Hydrierung von Olefinen der allgemeinen Formel VIII wird in an sich bekannter Weise, vorzugsweise mit katalytisch aktiviertem oder nascierendem Wasserstoff, wie Wasserstoff in Gegenwart von Kobalt-, Palladium-, Platin- oder Rhodium-Katalysatoren, z. B. Kobaltsulfid oder tris-(Triphenylphosphin)-rhodium-chlorid (welche durch Schwefel nicht vergiftet sind), oder mit elektrolytisch erzeugtem Wasserstoff, durchgeführt.

Ein weiteres Verfahren zur Herstellung der neuen Verbindungen besteht darin, daß man

e) eine Verbindung der allgemeinen Formel IX

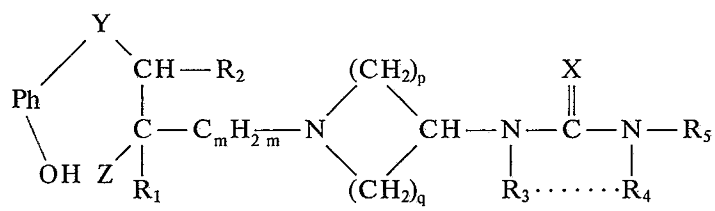

(IX)

worin Z eine reaktionsfähige veresterte Hydroxygruppe bedeutet, ringschließt.

Eine reaktionsfähige veresterte Hydroxygruppe ist z. B. eine mit einer starken anorganischen oder organischen Säure, vor allem einer Halogenwasserstoffsäure, z. B. Chlorwasserstoffsäure, Bromwasserstoffsäure oder Jodwasserstoffsäure, Schwefelsäure oder einer aromatischen Sulfonsäure, z. B. p-Toluolsulfonsäure oder m-Brombenzolsulfonsäure veresterte Hydroxygruppe. Die Kondensation wird vorzugsweise in Gegenwart von basischen Kondensationsmitteln, z. B.

Alkalimetall- oder Erdalkalimetall-hydroxyden, -carbonaten oder -hydrogencarbonaten, wie Natrium-, Kalium- oder Calcium-hydroxyd oder -carbonat, Alkalimetallhydriden, -niederalkoxiden oder -niederalkanoaten, wie Natriumhydrid, Natriummethylat oder Natriumacetat, oder von organischen tertiären Stickstoffbasen, wie Triniederalkylaminen oder Pyridinen, z. B. Triäthylamin oder Lutidin, durchgeführt.

Die erfindungsgemäß erhaltenen Verbindungen können in an sich bekannter Weise ineinander übergeführt werden. So können z. B. erhaltene Verbindungen, in welchen Y ein Schwefelatom bedeutet, mit milden Oxydationsmitteln zu ihren 4-Oxyden oxydiert werden. Man verwendet z. B. Perjodate, wie Natriumperjodat, in den genannten polaren Lösungsmitteln und arbeitet bei niedrigen Temperaturen, z. B. zwischen ungefähr 0° C und Zimmertemperatur. Man muß vorsichtig arbeiten, um einer Überoxydation aufgrund zu langer Reaktionsdauer vorzubeugen.

Schließlich können die Verbindungen der Erfindung in Form von freien Basen oder als Salze erhalten werden. Eine erhaltene freie Base kann in das entsprechende Säureadditionssalz, vorzugsweise mit Säuren, die therapeutisch verwendbare Säureadditionssalze ergeben, oder mit Anionenaustauschern, übergeführt werden. Erhaltene Salze können in die entsprechenden freien Basen, z. B. durch Behandlung mit einer stärkeren Base, wie mit einem Metallhydroxyd oder Ammoniumhydroxyd, basischem Salz oder einem Kationenaustauscher, z. B. mit einem Alkalimetallhydroxyd oder -carbonat, umgewandelt werden.

Säuren, die therapeutisch verwendbare Säureadditionssalze ergeben, sind z. B. anorganische Säuren, wie Halogenwasserstoffsäure, z. B. Chlorwasserstoff- oder Bromwasserstoffsäure, oder Schwefel-, Phosphor-, Salpeter- oder Perchlorsäure; oder organische Säuren, wie aliphatische oder aromatische Carbon- oder Sulfonsäuren, z. B. Ameisen-, Essig-, Propion-, Bernstein-, Glykol, Milch-, Äpfel-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl, Pamoe-, Nikotin-, Methansulfon-, Äthansulfon-, Hydroxyäthansulfon-, Äthylensulfon-, Halogenbenzolsulfon-, Toluolsulfon-, Naphthalinsulfon-, Sulfanil- oder Cyclohexylsulfaminsäure; oder die Ascorbinsäure. Diese oder andere Salze, z. B. die Pikrate, können auch in der Reinigung von freien Basen verwendet werden. Die Basen werden in ihre Salze übergeführt, die Salze abgetrennt und die Basen aus den Salzen freigesetzt.

Infolge der engen Beziehungen zwischen den neuen Verbindungen in freier Form und in Form ihrer Salze sind im vorausgegangenen und nachfolgend unter freien Verbindungen und Salzen sinn- und zweckgemäß gegebenenfalls auch die entsprechenden Salze bzw. freien Verbindungen zu verstehen.

Die Ausgangsstoffe sind bekannt oder, falls neu, können sie nach an sich bekannten Methoden, z. B. wie in den Beispielen beschrieben, hergestellt werden.

Verbindungen der Formel III können leicht durch Reduktion einer entsprechenden 1,4-Benzodioxan-2-yl-alkansäure zum entsprechenden Alkohol und Überführung der Hydroxygruppe in eine reaktionsfähige veresterte Hydroxygruppe, hergestellt werden. Die Reduktion wird mit Lithiumaluminiumhydrid oder Natrium-2-methoxyäthoxy-aluminiumhydrid und die Veresterung mit einer obengenannten starken Säure oder ihren Derivaten, z. B. mit einem Thionyl-, Phosphor- oder Benzolsulfonylhalogenid, in einem organischen Lösungsmittel, z. B. Benzol, vorzugsweise bei erhöhter Temperatur vorgenommen.

Bei der Herstellung von Verbindungen der Formel IV geht man von entsprechenden 1-Benzyl-piperidonen aus und wandelt diese in ihre O-Alkyloxime oder Schiff'sche Basen von Diaminoalkanen oder -benzolen um. Diese werden mit katalytisch aktiviertem oder nascierendem Wasserstoff zu den Mono- oder Diaminen reduziert. Zur Reduktion verwendet man z. B. Wasserstoff in Gegenwart von Kobalt-, Palladium-, Platin- oder Rhodium-Katalysatoren, z. B. Kobaltsulfid oder tris-(Triphenylphosphin)-rhodiumchlorid (welche durch Schwefel nicht vergiftet sind), oder elektrolytisch erzeugten Wasserstoff, oder einfache oder komplexe Leichtmetallhydride, z. B. Diboran oder Alan, Alkalimetallborhydride, -aluminiumhydride oder -alkoxyhydride, z. B. Lithiumaluminiumhydrid und/oder Natrium-trimethoxy-borhydrid. Die erhaltenen Amine werden weiter mit dem Kohlensäurederivat $Z_2CX'$, z. B. mit Ammoniumcyanat oder einem Metallcyanat oder -thiocyanat, Niederalkyl-isoharnstoff oder -isothioharnstoff, Halogencyan oder Cyanamid, Schwefelkohlenstoff oder Carbonylsulfid, Kohlensäurehalogenid oder 1,1-Carbonyldiimidazol, mit der Maßgabe, daß mindestens einer der Reste $Z_1$ und $Z_2$ Stickstoff enthält, umgesetzt. Schließlich wird die Benzylgruppe vorzugsweise über einem Palladium-Katalysator, hydrogenolytisch abgespalten.

Verbindungen der Formel V werden in analoger Weise hergestellt. Man setzt zuerst das 3- oder 4-Piperidon mit einem reaktionsfähigen Ester der Formel III um. Im reaktionsfähigen Ester ist die Hydroxygruppe z. B. durch eine starke anorganische oder organische Säure, in erster Linie eine Halogenwasserstoffsäure, z. B. Chlorwasserstoff-, Bromwasserstoff- oder Jodwasserstoffsäure, Schwefelsäure oder eine aromatische Sulfonsäure, wie p-Toluolsulfonsäure oder m-Brombenzolsulfonsäure verestert. Die erhaltenen Verbindungen werden in ihre O-Alkyloxime oder Schiff'sche Basen von Diaminoalkanen oder -benzolen umgewandelt. Diese werden mit katalytisch aktiviertem oder nascierendem Wasserstoff zu den Mono- oder Diaminen reduziert. Die Reduktion wird gleich wie bei der Herstellung von Ausgangsstofen der Formel IV beschrieben durchgeführt. Verbindungen der Formel V können auch durch Kondensation der vorher genannten Ester der Formel III mit

entsprechenden 3- oder 4-Benzylamino-piperidinen und Abspaltung der Benzylgruppe erhalten werden.

Verbindungen der Formel VII können ausgehend von den genannten 1,4-Benzodioxan-2-yl-alkansäuren hergestellt werden. Diese Säuren werden zuerst in ihre Halogenide, gemischte Anhydride oder Amide von Imidazol umgewandelt und dann mit entsprechenden Piperidinen umgesetzt.

Die ungesättigten Verbindungen der Formel VIII sind vorzugsweise Enamine, welche ausgehend von entsprechenden Aldehyden und bereits genannten Piperidinen hergestellt werden. Die Aldehyde können durch Reduktion der obengenannten Säurechloride nach Rosenmund oder durch Reduktion der entsprechenden Nitrile mit Diisobutylaluminiumhydrid erhalten werden.

Schließlich können die Verbindungen der Formel IX durch Mannich-Reaktion der genannten Piperidine mit entsprechenden Aldehyden und/oder Ketonen, Bromierung der erhaltenen Piperidino-alkanone, Kondensation der $\alpha$-Bromketone mit Monoacetyl-brenzcatechin, Reduktion des durch Kondensation erhaltenen Ketons mit Natriumborhydrid zum entsprechenden Alkohol und Umwandlung seiner Hydroxygruppe in eine reaktionsfähige, wie oben beschrieben, hergestellt werden.

Ausgangsstoffe und Endprodukte der Formeln I bis IX, welche Isomerengemische sind, können nach an sich bekannten Methoden, z. B. durch fraktionierte Destillation, Kristallisation und/oder Chromatographie, in die einzelnen Isomeren getrennt werden. Racemische Produkte können in die optischen Antipoden, z. B. bei Trennung ihrer diastereomeren Salze, z. B. durch fraktionierte Kristallisation der d- oder l-Tartrate, getrennt werden.

Die obengenannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder Neutralisationsmitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden oder das Verfahren auf irgendeiner Stufe abgebrochen wird oder wonach ein Ausgangsmaterial unter den Reaktionsbedingungen gebildet oder worin ein Ausgangsstoff in Form eines Salzes oder eines optisch reinen Antipoden verwendet wird.

Beim Verfahren der vorliegenden Erfindung werden vorzugsweise solche Ausgangsstoffe verwendet, welche zu den eingangs als besonders wertvoll geschilderten Verbindungen, insbesondere solchen der Formel II, führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z. B. Laktose, Dextrose, Rohrzucker, Mannitol, Sorbitol, Cellulose und/oder Glycin, und Schmiermitteln, z. B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, Enzyme der Bindemittel und/oder Brausemischungen, oder Adsorptionsmittel, Farbstoffe, Geschmacksstoffe und Süßmittel. Injizierbare Präparate sind vorzugsweise isotonische wässerige Lösungen oder Suspensionen und Suppositorien in erster Linie Fettemulsionen oder -suspensionen. Die pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 0,1% bis etwa 75%, insbesondere von etwa 1% bis etwa 50% des Aktivstoffes.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z. B. zwischen ungefähr 15 und 100 mm Hg, durchgeführt.

## Beispiel 1

Ein Gemisch von 4,9 g 2-(2-Tosyloxyäthyl)-1,4-benzodioxan, 2,54 g 1-(4-Piperidyl)-2-imidazolidinon, 5 g wasserfreiem Natriumcarbonat und 100 ml 4-Methyl-2-pentanon wird unter Rühren 3 Tage unter Rückfluß gekocht. Das Reaktionsgemisch wird filtriert, eingedampft und der Rückstand aus

Isopropanol umkristallisiert. Man erhält das 1-[1-[2-(1,4-Benzodioxan-2-yl)-äthyl]-4-piperidyl]-2-imidazolidinon der Formel

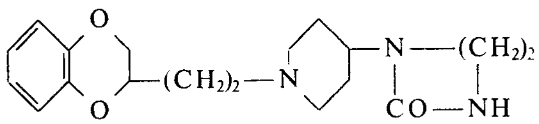

welches bei 125° schmilzt.

Das Produkt wird in 10 ml heißem Isopropanol suspendiert, die Suspension mit 5-normaler Bromwasserstoffsäure in Isopropanol neutralisiert und der Niederschlag aus Isopropanol umkristallisiert. Man erhält das entsprechende Hydrobromid, welches bei 220–221° unter Zersetzung schmilzt.

Der Ausgangsstoff wird wie folgt hergestellt: Eine Lösung von 6,7 g Allylcyanid in 30 ml Petroläther wird unter Rühren bei −15°, mit einer Lösung von 16 g Brom in 10 ml Petroläther langsam versetzt. Nach Abdampfen des Lösungsmittels erhält man das ölige 3,4-Dibrom-butyronitril in quantitativer Ausbeute.

In fünf gleichen Teilen gibt man tropfenweise insgesamt 227 g der letztgenannten Verbindung zu einem gerührten Gemisch von 85 g Brenzcatechin und 50 g wasserfreiem Kaliumcarbonat in 100 ml Aceton unter Rückfluß. Nach der Zugabe des ersten Teils werden weitere 50 g Kaliumcarbonat dazugegeben, und dann gibt man langsam den zweiten Teil des Nitrils dazu. Dies wiederholt man dreimal, wobei man aber je 40 g Kaliumcarbonat und einen Teil Nitril verwendet. Das Reaktionsgemisch wird dabei mit Aceton verdünnt, um das Rühren zu erleichtern. Dann läßt man das Gemisch 20 Stunden unter Rückfluß kochen und filtriert es. Das Filtrat wird eingedampft, der Rückstand destilliert und die bei 105°/0,15 mm Hg siedende Fraktion aufgefangen. Man erhält das 1,4-Benzodioxan-2-yl-acetonitril.

Ein Gemisch von 111 g der letztgenannten Verbindung, 63,5 ml Schwefelsäure, 160 ml Essigsäure und 160 ml Wasser wird 48 Stunden unter Rückfluß gekocht. Das Reaktionsgemisch wird auf Eis gegossen, das erhaltene feste Material abgetrennt und aus Benzol-Petroläther umkristallisiert. Man erhält das 1,4-Benzodioxan-2-yl-essigsäure, die bei 100° schmilzt.

Man läßt 16,5 ml einer 70%igen Lösung von Natrium-bis-(2-methoxy-äthoxy)-aluminiumhydrid in Benzol, in einer Stickstoffatmosphäre, unter Rückfluß kochen und versetzt sie tropfenweise mit einer Lösung von 5,8 g 1,4-Benzodioxan-2-yl-essigsäure in 100 ml Benzol. Nach der beendeten Zugabe wird das Gemisch 4 Stunden unter Rückfluß gekocht, abgekühlt und langsam in 20 ml 25%iger Schwefelsäure gegossen. Das Gemisch wird filtriert und das Lösungsmittel abgedampft. Der Rückstand wird in Methylenchlorid aufgenommen, die Lösung mit einer gesättigten wässerigen Natriumbicarbonatlösung gewaschen, getrocknet und eingedampft. Man erhält das ölige 2-(2-Hydroxyäthyl)-1,4-benzodioxan.

Ein Gemisch von 3,6 g der letztgenannten Verbindung, 5,7 g p-Toluolsulfonylchlorid und 20 ml trockenem Pyridin wird 2 Stunden unter Rühren in einem Eisbad gekühlt. Das Gemisch wird dann mit Eis versetzt, das erhaltene feste Material abfiltriert und aus Essigsäureäthylester-Petroläther umkristallisiert. Man erhält das 2-(2-Tosyloxyäthyl)-1,4-benzodioxan, welches bei 82–83° schmilzt.

Eine Lösung von 1,6 g 4-Aminopyridin in 7 ml Dimethylformamid wird unter Rühren unter 40°, mit 2 g 2-Chloräthylisocyanat versetzt. Das Gemisch wird nach 2 Stunden mit 28 ml Wasser versetzt und das Rühren bei Zimmertemperatur 2 Stunden fortgesetzt. Der erhaltene Niederschlag wird abfiltriert, mit Wasser gewaschen, getrocknet und aus wässerigem Äthanol umkristallisiert. Man erhält den 1-(4-Pyridyl)-3-(2-chloräthyl)-harnstoff, der bei 120–122° schmilzt.

Eine Suspension von 2,66 g der letztgenannten Verbindung in 4 ml siedendem Methanol wird unter Rühren, mit 2,68 g einer 30,8%igen Lösung von Natriummethylat in Methanol versetzt und das Gemisch 1 Stunde unter Rückfluß gerührt. Es wird heiß filtriert, mit heißem Methanol gewaschen, das Filtrat eingedampft und der Rückstand aus 90%igem wässerigen Äthanol umkristallisiert. Man erhält das 1-(4-Pyridyl)-2-imidazolidinon, welches bei 204–207° schmilzt.

Eine Lösung von 5 g der letztgenannten Verbindung in 45 ml Wasser wird über 0,8 g 10%igem Ruthenium-auf-Kohle Katalysator bei 120° und 120 Atmosphären bis zum Aufhören der Wasserstoffaufnahme hydriert. Das Reaktionsgemisch wird filtriert, das Filtrat eingedampft, der Rückstand in Chloroform aufgenommen, die Lösung getrocknet und eingedampft. Der Rückstand wird aus Methylenchlorid-Petroläther umkristallisiert. Man erhält das 1-(4-Piperidyl)-2-imidazolidinon, welches bei 155–157° schmilzt.

## Beispiel 2

Eine Lösung von 4 g 1-[1-[2-(I-1,4-Benzodioxan-2-yl)-acetyl]-4-piperidyl]-2-imidazolidinon in 50 ml Tetrahydrofuran wird bei 25° zu einer Suspension von 1 g Lithiumaluminiumhydrid in 100 ml Tetrahydrofuran unter Rühren und Kühlen gegeben. Das Reaktionsgemisch wird bei Zimmertemperatur über Nacht gerührt und mit 10 ml Essigsäureäthylester, 1 ml Wasser, 2 ml einer 15%igen

wässerigen Natriumhydroxydlösung und 3 ml Wasser zersetzt. Das Gemisch wird filtriert, das Filtrat eingedampft und der Rückstand in 25 ml heißem Isopropanol gelöst. Die Lösung wird mit 1,35 g Fumarsäure in 15 ml heißem Isopropanol versetzt und nach Abkühlen der erhaltene Niederschlag abgetrennt. Man erhält das l-1-[1-[-2-(1,4-Benzodioxan-2-yl)-äthyl-]-4-piperidyl]-2-imidazolidinon-fumarat, welches bei 210–213° schmilzt. $[\alpha]_D = -30°$ (Methanol).

In gleicher Weise wird auch das rechtsdrehende Salz erhalten, das bei 210–213° schmilzt. $[\alpha]_D = +30{,}8°$ (Methanol).

Der Ausgangsstoff wird wie folgt hergestellt: Man löst 19,4 g 1,4-Benzodioxan-2-yl-essigsäure und 12,1 g d-$\alpha$-Methyl-benzylamin in 100 ml heißem Isopropanol. Man läßt die Lösung über Nacht stehen, filtriert das erhaltene Salz ab und kristallisiert es aus Isopropanol fünfmal um. Die Versuche zeigen, daß dies zur optischen Trennung der genannten Säure genügt. Die Säure wird mit verdünnter Chlorwasserstoffsäure freigesetzt, das Gemisch mit Diäthyläther extrahiert und der Extrakt eingedampft. Man erhält die d-1,4-Benzodioxan-2-yl-essigsäure. $[\alpha]_D = +49°$ (Äthanol).

In analoger Weise, unter Verwendung des l-$\alpha$-Methyl-benzylamins, erhält man den anderen Säureantipoden. $[\alpha]_D = -49°$ (Äthanol).

Eine Lösung von 3,4 g der genannten l-Säure in 60 ml Tetrahydrofuran wird eine Stunde mit 3,4 g Carbonyldiimidazol, gerührt. Dann gibt man eine Suspension von 3,05 g 1-(4-Piperidyl)-2-imidazolidinon in 20 ml Tetrahydrofuran dazu und das Gemisch rührt man über Nacht. Es wird eingedampft, der Rückstand in Essigsäureäthylester gelöst, die Lösung mit normaler Chlorwasserstoffsäure und 5%iger wässeriger Natriumhydroxydlösung gewaschen, getrocknet und eingedampft. Man erhält das 1-[1-[2-(l-1,4-Benzodioxan-2-yl)-acetyl]-4-piperidyl]-2-imidazolidinon.

## Beispiel 3

Ein Gemisch von 3,34 g 2-(2-Tosyloxyäthyl)-1,4-benzodioxan, 1,83 g 1-(4-Piperidyl)-2-hexahydropyrimidinon, 4 g Natriumcarbonat und 80 ml 4-Methyl-2-pentanon wird 3 Tage unter Rückfluß gekocht. Das heiße Reaktionsgemisch wird abfiltriert, das Filtrat eingedampft und der Rückstand in Wasser aufgenommen. Die Suspension wird mit Ammoniumhydroxyd basisch gemacht, mit Chloroform extrahiert, der Extrakt getrocknet und eingedampft. Der Rückstand wird in heißem Äthanol gelöst, die Lösung mit 4,5-normaler Chlorwasserstoffsäure in Äthanol angesäuert, gekühlt und filtriert. Man erhält das 1-[1-[2-(1,4-Benzodioxan-2-yl)-äthyl]-4-piperidyl]-2-hexahydropyrimidinon-hydrochlorid, welches bei 253–255° schmilzt.

In analoger Weise wird auch das 1-[1-[2-(1,4-Benzodioxan-2-yl)-äthyl]-4-piperidyl]-2-benzimidazolidinon-hydrochlorid erhalten. Es schmilzt bei 185–189°.

Der Ausgangsstoff wird wie folgt hergestellt: Eine Lösung von 62,5 ml 1,3-Diaminopropan in 100 ml Äthanol wird unter Rühren und Kühlen mit Eis, mit 30 g 1-Benzyl-4-piperidon tropfenweise versetzt. Das Gemisch wird über 2 g vorreduziertem Platinoxyd bei 50° und 2,7 Atmosphären 9 Stunden hydriert. Nach der Aufnahme der theoretischen Wasserstoffmenge wird der Katalysator abfiltriert, das Filtrat eingedampft und der Rückstand destilliert. Die bei 145–160°/0,2 mm Hg siedende Fraktion wird aufgefangen. Man erhält das 4-(3-Amino-propylamino)-1-benzyl-piperidin.

Eine Lösung von 24,1 g der letztgenannten Verbindung in 100 ml Tetrahydrofuran wird unter Rühren und Kühlen mit Eis, mit 18,3 g 1,1-Carbonyldiimidazol in 250 ml Tetrahydrofuran tropfenweise versetzt. Das Reaktionsgemisch wird bei Zimmertemperatur 18 Stunden gerührt, eingedampft, der Rückstand in Wasser suspendiert, filtriert und aus Äthanol umkristallisiert. Man erhält das 1-(1-Benzyl-4-piperidyl)-2-hexahydropyrimidinon, welches bei 178–180° schmilzt.

Eine Lösung von 8 g der letztgenannten Verbindung in 100 ml Äthanol-Essigsäure (1 : 1) wird über 1,5 g 10%igem Palladium-auf-Kohle Katalysator 4 Stunden bei 50° und 2,7 Atmosphären hydriert. Das Reaktionsgemisch wird durch ein Filterelement filtriert, das Lösungsmittel abgedampft und der Rückstand in Wasser aufgenommen. Das Gemisch wird mit 50%iger wässeriger Natriumhydroxydlösung stark alkalisch gemacht, mit Chloroform extrahiert, der Extrakt getrocknet, filtriert und eingedampft. Der Rückstand wird aus Äthanol umkristallisiert. Man erhält das 1-(4-Piperidyl)-2-hexahydropyrimidinon, welches bei 206–210° schmilzt.

Das 1-(4-Piperidyl)-2-benzimidazolidinon ist im US-Patent 3 929 801 beschrieben.

## Beispiel 4

Eine Lösung von 6 g 1-[2-(1,4-Benzodioxan-2-yl)-äthyl]-4-(2-amino-äthylamino)-piperidin in 10 ml 50%igem wässerigem Äthanol wird bei 25° mit 1,4 ml Schwefelkohlenstoff tropfenweise versetzt. Das Gemisch wird eine Stunde unter Rückfluß gekocht, mit einem Tropfen konzentrierter Chlorwasserstoffsäure versetzt und 5 Stunden unter Rückfluß weiter gekocht. Man läßt das Reaktionsgemisch über Nacht auskühlen, filtriert es und wäscht den Rückstand mit Äthanol. Man erhält das 1-[1-[2-(1,4-Benzodioxan-2-yl)-äthyl]-4-piperidyl]-2-imidazolidinthion-hydrochlorid, welches bei 292° schmilzt.

Der Ausgangsstoff wird wie folgt hergestellt: Ein Gemisch von 10 g 2-(2-Tosyloxyäthyl)-1,4-benzodioxan, 10 g 4-Piperidon-hydrochlorid, 20 g wasserfreiem Natriumcarbonat und 160 ml Dimethylformamid wird bei Zimmertemperatur 48 Stunden stark gerührt. Das Reaktionsgemisch wird filtriert, der Rückstand mit einer geringen Menge Dimethylformamid gewaschen und das Filtrat eingedampft. Der Rückstand wird in Essigsäureäthylester gelöst, die Lösung mit Chlorwasserstoffsäure extrahiert, der Extrakt mit 50%iger wässeriger Natriumhydroxydlösung alkalisch gemacht und mit Methylenchlorid extrahiert. Der letztgenannte Extrakt wird getrocknet und eingedampft. Man erhält das 2-[2-(4-Oxopiperidino)-äthyl]-1,4-benzodioxan, welches sich beim Stehenlassen verfestigt.

Eine Lösung von 18 g der letztgenannten Verbindung in 150 ml Äthanol wird mit einer Lösung von 23 ml Äthylendiamin in 30 ml Äthanol versetzt und das Ganze über 2 g vorhydriertem Platinoxyd bei 50° und 3 Atmosphären bis zur Aufnahme der erforderlichen Menge Wasserstoff hydriert. Das Gemisch wird gekühlt, filtriert und das Filtrat eingedampft. Man erhält das 1-[2-(1,4-Benzodioxan-2-yl)-äthyl]-4-(2-amino-äthylamino)-piperidin als ein Öl.

## Beispiel 5

Gemäß den in den vorhergehenden Beispielen beschriebenen Methoden, vorzugsweise derjenigen der in der Tabelle durch »Bsp« bezeichneten Beispiele, werden auch die folgenden Verbindungen der Formel I, ausgehend von äquivalenten Mengen entsprechender Ausgangsstoffe hergestellt, In den Verbindungen der Tabelle ist $R_1 = R_2 = H, (R_3 + R_4) = (CH_2)_2$, X=0 und p=q=2. (Die Stellung 2 im Rest Ph ist bei Y).

| Nr. | Ph | Y | m | $R_5$ | Salz | Bsp. | F°C |
|---|---|---|---|---|---|---|---|
| 1 | 1.2-phenylene | 0 | 1 | H | HCl | 1 | 250–1 |
| 2 | 1,2-phenylene | 0 | 3 | H | HCl | 2 | 280–5 |
| 3 | 1,2-phenylene | 0 | 2 | phenyl | HCl | 1 | 263–5 |
| 4 | $4\text{-}CH_3\text{-}C_6H_3$ | 0 | 2 | H | HCl | 1 | 245–6 |
| 5 | $5\text{-}CH_3\text{-}C_6H_3$ | 0 | 2 | H | HCl | 1 | 218–0 |
| 6 | $6\text{-}CH_3O\text{-}C_6H_3$ | 0 | 2 | H | HCl | 1 | 261–1 |
| 7 | 1,2-phenylene | S | 2 | H | – | 2 | 95–9 |

Die verschiedenen Ausgangsstoffe werden wie folgt hergestellt:

Ein Gemisch von 10 g 2-(2-Tosyloxyäthyl)-1,4-benzodioxan, 2,4 g Natriumcyanid, 4 ml Wasser und 20 ml Äthanol wird 48 Stunden unter Rückfluß gekocht. Das Reaktionsgemisch wird eingedampft, der Rückstand in Wasser aufgenommen und mit Äther extrahiert. Der Extrakt wird getrocknet, eingedampft und 5 g des rohen Nitrils 48 Stunden in einem Gemisch von 2,8 ml Schwefelsäure, 7,2 ml Wasser und 7,2 ml Essigsäure, unter Rühren, unter Rückfluß gekocht. Das Reaktionsgemisch wird in Eiswasser gegossen, mit Diäthyläther extrahiert, der Extrakt mit Wasser gewaschen und mit wässeriger Natriumhydrogencarbonatlösung reextrahiert. Die alkalische Lösung wird mir Chlorwasserstoffsäure angesäuert und mit Diäthyläther extrahiert. Der Extrakt wird getrocknet und eingedampft. Man löst 2,5 g der rohen Säure in 25 ml Tetrahydrofuran und behandelt die Lösung mit 3 g Carbonyldiimidazol 30 Minuten unter Rühren. Das Gemisch wird mit 2,4 g 1-(4-Piperidyl)-2-imidazolidinon versetzt und über Nacht gerührt. Es wird eingedampft, der Rückstand in Essigsäureäthylester gelöst, die Lösung mit 5%iger wässeriger Natriumhydroxydlösung und 5%iger Chlorwasserstoffsäure gewaschen, getrocknet und eingedampft. Man erhält das 1-[1-[3-(1,4-Benzodioxan-2-yl)-propionyl]-4-piperidyl]-2-imidazolidinon.

Eine Lösung von 12,6 g N-Phenyl-äthylendiamin in 200 ml Methanol wird tropfenweise zuerst mit 50 ml 4,1-normaler Chlorwasserstoffsäure und dann mit 18,9 g 1-Benzyl-4-piperidon in 100 ml Methanol versetzt. Es werden dann 9,45 g Natriumcyanborhydrid portionenweise, unter Rühren bei Zimmertemperatur, dazu gegeben. Nach 72 Stunden wird das Gemisch filtriert, der Rückstand in Wasser gelöst und die Lösung mit 12,5%iger wässeriger Natriumhydroxydlösung basisch gemacht. Das Gemisch wird mit Methylenchlorid extrahiert, der Extrakt getrocknet und eingedampft. Man erhält das 1-Benzyl-4-(2-phenylamino-äthylamino)-piperidin.

Eine Lösung von 14 g der letztgenannten Verbindung in 100 ml trockenem Benzol wird unter Rühren bei Zimmertemperatur mit 170 ml 12,5%igem Phosgen in Benzol tropfenweise versetzt. Das Reaktionsgemisch wird über Nacht gerührt, der gelatinöse Niederschlag mit einem Sinterglastrichter

filtriert und das Material in heißem Wasser aufgelöst. Die Lösung wird mit Ammoniumhydroxyd basisch gemacht und der Niederschlag abfiltriert. Man erhält das 1-(1-Benzyl-4-piperidyl)-3-phenyl-2-imidazolidinon, welches bei 168—170° schmilzt.

Eine Lösung von 10 g der letztgenannten Verbindung in 200 ml Äthanol-Essigsäure (1 : 1) wird über 1 g 10%igem Palladium-auf-Kohle Katalysator 8 Stunden bei 50° und 2,7 Atmosphären hydriert. Das Reaktionsgemisch wird durch ein Filterelement filtriert und das Lösungsmittel abgedampft. Der Rückstand wird mit 25%iger wässeriger Natriumhydroxydlösung basisch gemacht und das Gemisch mit Diäthyläther extrahiert. Die organische Schicht wird getrocknet und eingedampft. Man erhält das 1-(4-Piperidyl)-3-phenyl-2-imidazolidinon.

Eine Lösung von 48,6 g 2-(7-Methyl-1,4-benzodioxan-2-yl)-essigsäure in einer minimalen Menge Tetrahydrofuran wird tropfenweise zu einer unter Rückfluß kochenden, gerührten Suspension von 13,4 g Lithiumaluminiumhydrid in 200 ml trockenem Tetrahydrofuran gegeben. Das Gemisch wird über Nacht unter Rückfluß gekocht, abgekühlt und durch Zusetzen von 13,4 ml Wasser, 13,4 ml 15%iger wässeriger Natriumhydroxydlösung und 40 ml Wasser zersetzt. Das Gemisch wird filtriert, eingedampft, der Rückstand in einem Molekulardestillationsapparat destilliert und die bei 155—165°/0,1 mm Hg siedende Fraktion als farbloses Öl aufgefangen. Man erhält das 2-(2-Hydroxyäthyl)-7-methyl-1,4-benzodioxan.

Eine gerührte, unter Rückfluß kochende Lösung von 75 g 2-Hydroxythiophenol in 660 ml Aceton wird zuerst mit 42 g Kaliumcarbonat und dann tropfenweise mit einer Lösung von 33,5 g 3,4-Dibrombuttersäurenitril in 50 ml Aceton versetzt. Das Gemisch wird 30 Minuten unter Rückfluß gekocht und dann eine zweite, dritte und vierte Zugabe von 42 g Kaliumcarbonat und 33,5 g 3,4-Dibrombuttersäurenitril durchgeführt. Das Reaktionsgemisch wird 20 Stunden unter Rückfluß gekocht, gekühlt und filtriert. Das Filtrat wird eingedampft und der Rückstand in einem Kugelrohrapparat bei 185° und 0,5 mm Hg destilliert. Man erhält das 2-(1,4-Benzoxathian-2-yl)-acetonitril.

Ein Gemisch von 57 g der letztgenannten Verbindung, 85 ml Wasser, 85 ml Essigsäure und 32,6 ml Schwefelsäure wird 48 Stunden unter Rückfluß gekocht, abgekühlt, auf Eis gegossen und mit Benzol extrahiert. Die organische Phase wird mit wässeriger Natriumhydrogencarbonatlösung extrahiert, die wässerige Phase mit Chlorwasserstoffsäure angesäuert und mit Diäthyläther extrahiert. Der Extrakt wird getrocknet und eingedampft. Man erhält die entsprechende Carbonsäure als ein Öl. Diese Verbindung wird zuerst mit Carbonyldiimidazol und dann mit 1-(4-Piperidyl)-2-imidazolidinon gemäß Beispiel 2 umgesetzt, wobei man das gewünschte Amid erhält.

## Beispiel 6

Eine bei 5° gerührte Lösung von 6 g 1-[2-(1,4-Benzodioxan-2-yl)-äthyl]-4-(2-amino-äthylamino)-piperidin in 15 ml Tetrahydrofuran wird mit 1,58 g Bromcyan in 15 ml Tetrahydrofuran versetzt. Nach 2 Stunden wird das Gemisch filtriert und der Rückstand in einer minimalen Menge Wasser gelöst. Die Lösung wird mit Natriumhydroxyd stark basisch gemacht und mit Methylenchlorid extrahiert. Der Extrakt wird getrocknet, eingedampft und der Rückstand in 50 ml wasserfreiem Äthanol aufgenommen. Die Lösung wird mit 0,1 g Natriummethoxid versetzt. Das Gemisch wird über Nacht unter Rückfluß gekocht und eingedampft. Der Rückstand wird in Wasser aufgenommen und das Gemisch mit Methylenchlorid extrahiert. Der Extrakt wird getrocknet, eingedampft und der Rückstand in einer minimalen Menge Isopropanol aufgelöst. Die Lösung wird mit Oxalsäure in Diäthyläther neutralisiert und der erhaltene Niederschlag abgetrennt. Man erhält das 1-[1-[2-(1,4-Benzodioxan-2-yl)-äthyl]-4-piperidyl]-2-imino-imidazolidin-oxalat, welches bei 215—220° unter Zersetzung schmilzt.

## Beispiel 7

Herstellung von 10 000 Tabletten mit einem Gehalt von je 5 mg der aktiven Substanz

Bestandteile:

| | |
|---|---|
| 1-[1-[2-(1,4-Benzodioxan-2-yl)-äthyl]-4-piperidyl]-2-imidazolidinon | 50 g |
| Milchzucker | 1157 g |
| Maisstärke | 75 g |
| Polyäthylenglykol 6000 | 75 g |
| Talkpulver | 75 g |
| Magnesiumstearat | 18 g |
| gereinigtes Wasser | q. s. |

Verfahren:

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepreßt.

Herstellung von 10 000 Kapseln mit einem Gehalt von je 2,5 mg der aktiven Substanz

Bestandteile:

| | |
|---|---|
| 1-[1-[2-(1,4-Benzodioxan-2-yl)-äthyl]-4-piperidyl]-imidazolidinon | 25 g |
| Milchzucker | 1875 g |
| Talkpulver | 100 g |

Verfahren:

Sämtliche pulverigen Bestandteile werden mit einem Sieb von 0,6 mm Maschenweite gesiebt. Dann wird der Wirkstoff zuerst mit Talk und nachher mit Milchzucker in einem geeigneten Mischer homogenisiert. Gelatine Kapseln Nr. 3 werden mit je 200 mg der erhaltenen Mischung in einer Füllmaschine abgefüllt.

In analoger Weise werden auch Tabletten und harte Gelatine-Kapseln unter Verwendung von Produkten der anderen Beispiele, hergestellt.

Beispiel 8

Eine Lösung von 2 g 1-[1-[2-(1,4-Benzodioxan-2-yl)-äthyl]-4-piperidyl]-2-imidazolidinon in einer minimalen Menge Äthanol wird mit einer Lösung von 0,78 g Fumarsäure in siedendem Äthanol versetzt. Das Gemisch wird auf 0° gekühlt und der Niederschlag abgetrennt. Man erhält das entsprechende Fumarat, welches bei 190° schmilzt.

Beispiel 9

Eine Lösung von 3,47 g 1-[1-[2-(1,4-Benzoxathian-2-yl)-äthyl]-4-piperidyl]-2-imidazolidinon (Beispiel 5, Nr. 7) in 10 ml Dioxan und 10 ml Methanol wird unter Rühren bei Zimmertemperatur mit einer Lösung von 2,8 g Natriummetaperjodat in 20 ml Wasser tropfenweise versetzt. Nach 2 Stunden wird das Gemisch eingedampft, der Rückstand in Wasser aufgenommen und das Gemisch mit Methylenchlorid extrahiert. Der Extrakt wird mit gesättigter wässeriger Natriumchloridlösung gewaschen, getrocknet, eingedampft und der Rückstand aus Isopropanol umkristallisiert. Man erhält das 1-[1-[2-(4-Oxo-1,4-benzoxathian-2-yl)-äthyl]-4-piperidyl]-2-imidazolidinon, welches bei 145° schmilzt.

Beispiel 10

Ein Gemisch von 0,5 g 1-[1-[3-(1,4-Benzodioxan-2-yl)-1-propenyl]-4-piperidyl]-2-imidazolidinon, 25 ml Äthanol-Essigsäure (1 : 1) und 0,1 g 10%igem Palladium-auf-Kohle Katalysator wird bei Zimmertemperatur und 2,7 Atmosphären bis zur Aufnahme von einem Moläquivalent Wasserstoff hydriert. Der Katalysator wird abfiltriert, das Filtrat eingedampft, der Rückstand in 5 ml Wasser gelöst und die Lösung mit wässerigem Ammoniak gemacht. Das Gemisch wird mit Methylenchlorid extrahiert, der Extrakt getrocknet, eingedampft und der Rückstand in einer minimalen Menge Isopropanol aufgenommen. Die Lösung wird mit Chlorwasserstoff in Äthanol angesäuert und der Niederschlag abgetrennt. Man erhält das 1-[1-[3-(1,4-Benzodioxan-2-yl)-propyl]-4-piperidyl]-2-imidazolidinon-hydrochlorid, welches bei 282–285° unter Zersetzung schmilzt. Das Produkt ist mit demjenigen des Beispiels 5, Nr. 2 identisch.

Der Ausgangsstoff wird wie folgt hergestellt: Ein Gemisch von 10 g 2-(1,4-Benzodioxan-2-yl)-oxiran, 2 g Kaliumcyanid, 2 g Ammoniumchlorid und 25 ml Dimethylformamid wird 3 Tage bei Zimmertemperatur gerührt. Das Gemisch wird mit Wasser verdünnt und mit Methylenchlorid extrahiert. Der Extrakt wird eingedampft und der Rückstand in einem Gemisch von 11 ml Essigsäure, 11 ml Wasser und 4 ml Schwefelsäure, durch Kochen unter Rückfluß 24 Stunden, dehydriert. Das Gemisch wird mit Eis verdünnt, mit Diäthyläther extrahiert und der Extrakt eingedampft. Man erhält die 3-(1,4-Benzodioxan-2-yl)-acrylsäure. Eine Lösung von 5 g dieser Verbindung in 25 ml Tetrahydrofuran wird mit 5,5 g Carbonyldiimidazol behandelt und das Gemisch 30 Minuten gerührt. Dann wird eine Lösung von 4,6 g 1-(4-Piperidyl)-2-imidazolidinon in 25 ml Isopropanol dazu gegeben und das Gemisch über Nacht stehengelassen. Das Reaktionsgemisch wird eingedampft, der Rückstand in Essigsäure-Äthylester aufgenommen, die Lösung nacheinander mit Wasser, verdünnter wässeriger Natriumhydroxydlösung und Wasser gewaschen und eingedampft. Der Rückstand wird in 100 ml Tetrahydrofuran gelöst, im Eisbad gekühlt und tropfenweise mit 20 ml einer 1,2-molaren Lösung von Alan-Triäthylamin-Komplex unter Rühren behandelt. Nach 5 Stunden wird das kalte Gemisch tropfenweise mit 10 ml 25%iger wässeriger Natriumhydroxydlösung behandelt. Die organische Lösungsmittelphase wird durch Dekantieren vom pastenartigen Schlamm der anorganischen Salze abgetrennt und eingedampft. Man erhält das 1-[1-[3-(1,4-Benzodioxan-2-yl)-1-propenyl]-4-piperidyl]-2-imidazolidinon.

## Beispiel 11

Ein Gemisch von 4 g 2-(2-Tosyloxyäthyl)-6,7-dichlor-1,4-benzodioxan, 1,69 g 1-(4-Piperidyl)-2-imidazolidinon, 10 g wasserfreiem Natriumcarbonat und 100 ml 4-Methyl-2-pentanon wird unter Rühren 2 Tage unter Rückfluß gekocht. Das Reaktionsgemisch wird filtriert, das Filtrat eingedampft und der Rückstand aus Aceton umkristallisiert. Man erhält das 1-[1-[2-(6,7-Dichlor-1,4-benzodioxan-2-yl)-äthyl]-4-piperidyl]-2-imidazolidinon, welches bei 165° schmilzt.

Das Produkt wird in 10 ml heißem Äthanol suspendiert, die Suspension mit 5-normaler Chlorwasserstoffsäure in Äthanol neutralisiert und der Niederschlag aus Äthanol-Diäthyläther umkristallisiert. Man erhält das entsprechende Hydrochlorid, welches bei 250° schmilzt.

Der Ausgangsstoff wird wie folgt hergestellt: Eine Lösung von 60 g Brenzcatechin in 200 ml Diäthyläther wird unter Rühren langsam (in 2 Stunden) mit 93 g Sulfurylchlorid versetzt, wobei man die Temperatur bei ungefähr 0° hält. Man läßt das Reaktionsgemisch 2 Tage bei Zimmertemperatur stehen, dampft es ein und kristallisiert den Rückstand aus Benzol zweimal um. Man erhält das 4,5-Dichlor-brenzcatechin, welches bei 85–90° schmilzt. Zu einem Gemisch von 53,4 g der letztgenannten Verbindung, 33 g wasserfreiem Kaliumcarbonat und 800 ml Aceton gibt man tropfenweise unter Rühren und Kochen unter Rückfluß, in fünf gleichen Teilen insgesamt 89 g 3,4-Dibrom-butyronitril. Dann gibt man weitere 20 g Kaliumcarbonat und nachher langsam den zweiten Teil des Nitrils zum Reaktionsgemisch. Dies wiederholt man noch dreimal, wobei man auf einen Teil Nitril je 20 g Kaliumcarbonat verwendet und zur Erleichterung des Rührens das Gemisch mit Aceton verdünnt. Das Reaktionsgemisch wird 20 Stunden unter Rückfluß gekocht, filtriert, das Filtrat eingedampft, der Rückstand in Methylenchlorid aufgenommen, die Lösung mit Wasser gewaschen, getrocknet und eingedampft. Unter Verwendung von Aktivkohle wird der Rückstand aus Isopropanol umkristallisiert. Man erhält das 6,7-Dichlor-1,4-benzodioxan-2-yl-acetonitril, welches bei 110° schmilzt.

Ein Gemisch von 59 g der letztgenannten Verbindung, 24 ml Schwefelsäure, 60 ml Essigsäure und 60 ml Wasser wird 48 Stunden unter Rückfluß gekocht. Das Reaktionsgemisch wird auf Eis gegossen. Das erhaltene feste Material wird abgetrennt und aus wässerigem Äthanol umkristallisiert. Man erhält die 6,7-Dichlor-1,4-benzodioxan-2-yl-essigsäure, welche bei 145–147° schmilzt.

Eine Lösung von 2,63 g der letztgenannten Verbindung in 40 ml Benzol-Tetrahydrofuran (1 : 1) wird in einer Stickstoffatmosphäre tropfenweise zu 5,5 ml einer unter Rückfluß kochenden 70%igen Lösung von Natrium-bis(2-methoxy-äthoxy)-aluminiumhydrid gegeben. Nach der beendeten Zugabe wird das Gemisch 2,5 Stunden unter Rückfluß gekocht, abgekühlt und langsam in 6,7 ml 20%iger Schwefelsäure gegeben. Das Gemisch wird filtriert und das Lösungsmittel abgedampft. Der Rückstand wird in Methylenchlorid aufgenommen, die Lösung mehrmals mit gesättigter wässeriger Natriumhydrogencarbonatlösung, Wasser und gesättigter wässeriger Natriumchloridlösung gewaschen, getrocknet und eingedampft. Man erhält das ölige 2-(2-Hydroxyäthyl)-6,7-dichlor-1,4-benzodioxan, welches bei 180–190°/0,1 mm Hg siedet.

Ein Gemisch von 15,2 g der letztgenannten Verbindung, 17,5 g p-Toluolsulfonylchlorid und 40 ml trockenem Pyridin wird 2 Stunden in einem Eisbad gekühlt und gerührt. Das Reaktionsgemisch wird dann mit Eis versetzt, der erhaltene Niederschlag abfiltriert und aus Essigsäureäthylester umkristallisiert. Man erhält das 2-(2-Tosyloxyäthyl)-6,7-dichlor-1,4-benzodioxan, welches bei 135–138° schmilzt.

## Beispiel 12

Eine Suspension von 100 g Lithiumaluminiumhydrid in 6500 ml Tetrahydrofuran wird unter Rühren bei 2–6°, portionenweise, innerhalb von 100 Minuten, mit 450 g d-1-[1-[2-(l-1,4-benzodioxan-2-yl)-acetyl]-4-piperidyl]-2-imidazolidinon versetzt. Das Reaktionsgemisch wird 19 Stunden bei Zimmertemperatur gerührt, wieder gekühlt und zuerst mit 100 ml Wasser in 100 Minuten bei 8 – 12°, dann mit 100 ml 15%iger wässeriger Natriumhydroxydlösung und 300 ml Wasser versetzt. Das Gemisch wird 30 Minuten unter Kühlung und 90 Minuten bei Zimmertemperatur gerührt. Das Reaktionsgemisch wird abfiltriert, der Rückstand mit 2000 ml Tetrahydrofuran gewaschen und das Filtrat eingedampft. Man erhält das l-1-[1-[2-(1,4-Benzodioxan-2-yl]-äthyl]-4-piperidyl]-2-imidazolidinon, welches bei 119 – 122° schmilzt. $[\alpha]_D^{25} = -47,3°$ (c = 1, in Methanol).

Man löst 1191 g der erhaltenen Verbindung in 3000 ml 95%igem wässerigem Äthanol bei 60°, versetzt die Lösung mit 120 g Aktivkohle und rührt das Gemisch 5 Minuten. Es wird filtriert, der Rückstand mit 200 ml 95%igem Äthanol gewaschen und das Filtrat mit einer Lösung von 441 g Fumarsäure in 7200 ml 95%igem Äthanol bei 60° vereinigt. Die erhaltene Suspension wird mit 1000 ml 95%igem Äthanol verdünnt und 3 Stunden bei Zimmertemperatur gerührt. Das Gemisch wird filtriert, der Rückstand mit 500 ml 95%igem Äthanol und 800 ml wasserfreiem Diäthyläther gewaschen. Man erhält das l-1-[1-[2-(1,4-Benzodioxan-2-yl)-äthyl]-4-piperidyl]-2-imidazolidinon-fumarat, welches bei 216 – 217° unter Zersetzung schmilzt. $[\alpha]_D^{25} = -31,6°$ (c = 13,73 mg/ml, in Wasser). Das Produkt ist mit demjenigen des Beispiels 2 identisch.

Der Ausgangsstoff wird wie folgt hergestellt: Eine Lösung von 1134 g Brom in 1400 ml Essigsäureäthylester wird innerhalb 90 Minuten, unter Rühren bei –10 bis 0°, mit 472 g Allylcyanid tropfenweise versetzt. Die erhaltene Lösung von 3,4-Dibrom-butyronitril wird auf einmal, unter Rühren in einer Stickstoffatmosphäre bei 35°, zu einer Lösung, welche von 705 g Brenzcatechin und 1987 g wasserfreiem Kaliumcarbonat in 5000 ml unter Rückfluß kochendem Essigsäureäthylester hergestellt ist, gegeben. Das Gemisch wird 4 Stunden unter Rückfluß gekocht und über Nacht bei Zimmertemperatur gerührt. Es wird filtriert, der Rückstand mit 2000 ml Essigsäureäthylester gewaschen und das Filtrat eingedampft. Der Rückstand wird destilliert und die bei 138°/1,4 – 131°/1,1 mm siedende Fraktion aufgefangen. Man erhält das 1,4-Benzodioxan-2-yl-acetonitril.

Eine Lösung von 1647 g der letztgenannten Verbindung in 2760 ml Eisessig wird zum heißen Gemisch von 858 ml konzentrierter Schwefelsäure und 2670 ml Wasser gegeben und das ganze 17 Stunden unter Rückfluß gekocht. Das Gemisch wird in 8200 ml kaltes Wasser gegossen, 3 Stunden gerührt, filtriert und mit 9000 ml Wasser gewaschen. Man erhält die 1,4-Benzodioxan-2-yl-essigsäure, die bei 87 – 90° schmilzt.

Ein Gemisch von 3374 g der letztgenannten Verbindung und 3500 ml wasserfreiem Äthanol wird mit einer Lösung von 2105 g l-α-Methyl-benzylamin in 500 ml wasserfreiem Äthanol versetzt und das Gemisch 3 Stunden bei Zimmertemperatur gerührt. Man läßt das Gemisch bei 4 – 5° zwei Tage stehen, filtriert das erhaltene Salz ab und wäscht es mit 400 ml Äthanol, 400 ml Diäthyläther und 1200 ml Isopropanol. Man kristallisiert 2070 g des Niederschlags in 2000 ml Äthanol um und wäscht die Kristalle mit je 500 ml Äthanol und Diäthyläther. Man erhält das l-1,4-Benzodioxan-2-yl-essigsäure-l-α-methyl-benzylammoniumsalz, welches bei 132 – 133° schmilzt.

Man löst 1827 g der letztgenannten Verbindung in 10 000 ml normaler Chlorwasserstoffsäure unter Rühren für 20 Minuten, gibt 6000 ml Diäthyläther dazu und rührt es 20 Minuten weiter. Die wässerige Schicht wird abgetrennt, mit 3000 ml Diäthyläther extrahiert, die vereinigten ätherischen Lösungen werden mit 1000 ml Wasser gewaschen, getrocknet und eingedampft. Man erhält die l-1,4-Benzodioxan-2-yl-essigsäure, welche bei 83 – 85° schmilzt. $[\alpha]_D^{25} = -55,42°$ (c = 12,325 mg/ml, in Methanol).

Ein Gemisch von 400 g der letztgenannten Verbindung, 2000 ml Toluol und 320 ml Thionylchlorid wird 3 Stunden bei 80° gerührt und eingedampft. Der Rückstand wird in 500 ml Chloroform aufgenommen und die Lösung wieder eingedampft. Man erhält das l-1,4-Benzodioxan-2-yl-acetylchlorid, welches bei 60 – 62° schmilzt.

Eine Lösung von 434 g der letztgenannten Verbindung in 1000 ml Chloroform wird innerhalb 2 Stunden zu einem Gemisch von 400 g 1-(4-Piperidyl)-2-imidazolidinon, 4000 ml Chloroform und 2600 ml normaler wässeriger Natriumcarbonatlösung, unter Rühren bei 20 – 23° gegeben. Nach 2 Stunden wird die organische Lösung abgetrennt und die wässerige Phase mit 1000 ml Chloroform extrahiert. Die vereinigten organischen Lösungen werden mit 1000 ml Wasser gewaschen, getrocknet und eingedampft. Der Rückstand wird mit 1000 ml wasserfreiem Diäthyläther trituriert, filtriert und der Rückstand mit weiteren 1000 ml Diäthyläther gewaschen.

Man erhält das d-1-[1-[2-(l-1,4-Benzodioxan-2-yl)-acetyl]-4-piperidyl]-2-imidazolidinon, welches bei 161 – 163° schmilzt. $[\alpha]_D^{25} = +5,9°$ (c = 1, in Chloroform).

## Beispiel 13

Eine Lösung von 2 g 1-[2-(1,4-Benzodioxan-2-yl)-äthyl]-4-(2-aminoäthylamino)-piperidin in 25 ml Isopropanol wird unter Rühren zuerst mit 1,65 ml 4-normaler Chlorwasserstoffsäure in Äthanol und dann mit 0,45 g Ammoniumcyanat versetzt. Das Reaktionsgemisch wird über Nacht unter Rückfluß gekocht, dann eingedampft und in einem Ölbad 4 Stunden bei 130° C erhitzt. Der Rückstand wird in 100 ml Methylenchlorid gelöst, mit Wasser gewaschen, über wasserfreiem Natriumsulfat getrocknet

und eingedampft. Der Rückstand wird in 20 ml heißem Isopropanol gelöst und unter Rühren mit 0,77 g Fumarsäure versetzt. Beim Kühlen erhält man das Fumarat des 1-[1-[2-(1,4-Benzodioxan-2-yl)-äthyl]-4-piperidyl]-2-imidazolidinons, welches bei 190° C schmilzt.

Beispiel 14

Ein Gemisch von 10 g 1-(1,4-Benzodioxan-2-yl)-äthyl]-4-(2-amino-äthylamino)-piperidin und 8 g Carbonyl-diimidazol in 100 ml Tetrahydrofuran wird über Nacht unter Rückfluß gekocht. Das Reaktionsgemisch wird eingedampft, der Rückstand in 150 ml Methylenchlorid aufgenommen und dreimal mit 50 ml Wasser extrahiert. Das Methylenchlorid wird über wasserfreiem Natriumsulfat getrocknet und eingedampft. Der Rückstand wird in 50 ml heißem Isopropanol gelöst und unter Rühren mit 3,8 g Fumarsäure versetzt. Beim Kühlen erhält man das 1-[1-[2-(1,4-Benzodioxan-2-yl)-äthyl]-4-piperidyl]-2-imidazolidinon-fumarat, welches bei 190° C schmilzt.

Beispiel 15

Man löst 10 g 1-[1-[2-(1,4-Benzodioxan-2-yl)-äthyl]-4-(1,2,5,6-tetrahydropyridyl)]-2-imidazolidinon in 100 ml Essigsäure und 50 ml Wasser, gibt 1 g Platinoxyd dazu und hydriert das Gemisch bei 60° C und 3,4 Atmosphären bis zur Aufnahme von einem Moläquivalent Wasserstoff. Das Reaktionsgemisch wird auf Zimmertemperatur gekühlt, der Katalysator abfiltriert und das Filtrat eingedampft. Der Rückstand wird mit 100 ml Wasser, mit überschüssiger 3-normaler Natriumhydroxydlösung (bis zum pH-Wert 10) und 100 ml Methylenchlorid versetzt. Der Methylenchlorid-Extrakt wird mit Wasser gewaschen, über wasserfreiem Natriumsulfat getrocknet und eingedampft. Der Rückstand wird in 50 ml heißem Isopropanol aufgenommen und unter Rühren mit 3,8 g Fumarsäure behandelt. Beim Kühlen erhält man das Fumarsäuresalz des gewünschten 1-[1-[2-(1,4-Benzodioxan-2-yl)-äthyl]-4-piperidyl]-2-imidazolidinons, welches bei 190° C schmilzt.

Der Ausgangsstoff wird wie folgt hergestellt: Eine Lösung von 10 g 2-(2-Tosyloxyäthyl)-1,4-benzodioxan, 4,9 g 1-(4-Pyridyl)-2-imidazolidinon und 0,3 g Kaliumjodid wird über Nacht bei 90° C erhitzt. Das Reaktionsgemisch wird dann eingedampft. Der Rückstand wird in 500 ml Äthanol gelöst und unter Rühren und Kühlen innerhalb 2 Stunden mit 30 g Natriumborhydrid portionenweise behandelt. Das Reaktionsgemisch wird zu einem kleinen Volumen eingedampft, mit 100 ml Wasser verdünnt und mit 200 ml Methylenchlorid extrahiert.

Der Extrakt wird mit Wasser gewaschen, über wasserfreiem Natriumsulfat getrocknet und eingedampft. Man erhält das 1-[1-[2-(1,4-Benzodioxan-2-yl)-äthyl]-4-(1,2,5,6-tetrahydropyridyl)]-2-imidazolidinon, welches als Ausgangsstoff eingesetzt wird.

**Patentansprüche**

1. N-Oxacyclyl-alkyl-piperidyl-diazaverbindungen der allgemeinen Formel I

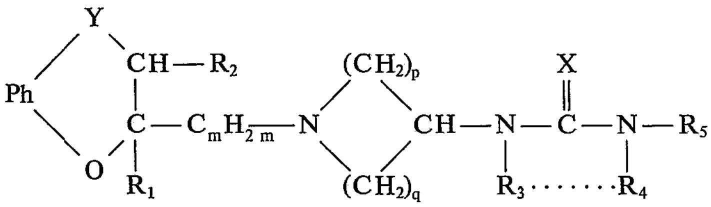

(I)

worin Ph unsubstituiertes 1,2-Phenylen oder 1,2-Phenylen, welches durch einen bis drei gleiche oder verschiedene Substituenten der Gruppe bestehend aus Niederalkyl, Niederalkoxy, Niederalkylendioxy, Halogen oder Trifluormethyl substituiert ist, bedeutet, jedes der Symbole $R_1$ und $R_2$ für Wasserstoff oder Niederalkyl steht, jedes der Symbole $R_3$ und $R_4$ Wasserstoff oder Niederalkyl bedeutet oder $(R_3+R_4)$ für Ph oder Niederalkylen steht, welches die beiden Stickstoffatome durch 2 bis 4 Kohlenstoffatome trennt, $R_5$ Wasserstoff, Niederalkyl oder HPh bedeutet, X für Oxo, Thioxo, Imino oder Niederalkylimino steht, Y Epoxy, Epithio oder Sulfinyl bedeutet, m für eine ganze Zahl von 1 bis 7 steht, jedes der Symbole p und q eine ganze Zahl von 1 bis 3 bedeutet, wobei $(p+q)$ die Zahl 4 ist, und ihre Säureadditionssalze.

2. Verbindungen nach Anspruch 1, Formel I, worin Ph unsubstituiertes oder durch Niederalkyl, Niederalkoxy, Niederalkylendioxy, Halogen oder Trifluormethyl monosubstituiertes 1,2-Phenylen bedeutet, jedes der Symbole $R_1$ und $R_2$ für Wasser oder Niederalkyl steht, jedes der Symbole $R_3$ und $R_4$ Wasserstoff oder Niederalkyl ist oder $(R_3+R_4)$ den Rest Ph oder Niederalkylen bedeutet, welches die beiden Stickstoffatome durch 2 oder 3 Kohlenstoffatome trennt, das Symbol $R_5$ für Wasserstoff,

Niederalkyl oder HPh steht, X Oxo, Thioxo, Imino oder Niederalkylimino bedeutet, Y für Epoxy, Epithio oder Sulfinyl steht, m für eine ganze Zahl von 1 bis 4 steht, jedes der Symbole p und q eine ganze Zahl von 1 bis 3 bedeutet, wobei (p+q) die Zahl 4 ist und ihre therapeutisch verwendbaren Säureadditionssalze.

3. Verbindungen nach Anspruch 1 der Formel I, worin Ph unsubstituiertes oder durch Alkyl oder Alkoxy, jeweils mit höchstens 4 Kohlenstoffatomen, Halogen oder Trifluormethyl monosubstituiertes 1,2-Phenylen bedeutet, jedes der Symbole $R_1$, $R_2$ und $R_5$ für Wasserstoff oder Alkyl mit 4 Kohlenstoffatomen steht, jedes der Symbole $R_3$ und $R_4$ Wasserstoff bedeutet oder $(R_3+R_4)$ für Alkylen mit 2 bis 4 Kohlenstoffatomen steht, welches die beiden Stickstoffatome durch 2 oder 3 Kohlenstoffatome trennt, X Oxo, Thioxo oder Imino bedeutet, Y für Epoxy oder Epithio steht, m für eine ganze Zahl von 1 bis 4 steht, jedes der Symbole p und q 2 bedeutet, und ihre therapeutisch verwendbaren Säureadditionssalze.

4. Verbindungen nach Anspruch 1, welche der allgemeinen Formel II

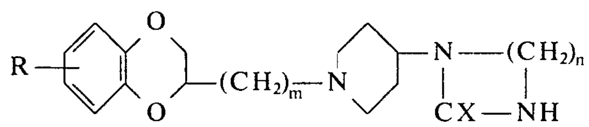

(II)

entsprechen, worin R Wasserstoff, Alkyl oder Alkoxy, jeweils mit höchstens 4 Kohlenstoffatomen, Halogen oder Trifluormethyl bedeutet, m für eine ganze Zahl von 1 bis 4 steht, n die ganze Zahl 2 oder 3 bedeutet, und X ist Oxo, Thioxo oder Imino, und ihre therapeutisch verwendbaren Säureadditionssalze.

5. Eine Verbindung nach Anspruch 1, welche das 1-[1-[2-(1,4-Benzoxathian-2yl)-äthyl]-4-piperidyl]-2-imidazolidinon ist und seine therapeutisch verwendbaren Säureadditionssalze.

6. Eine Verbindung gemäß Anspruch 4, welche das 1-[1-[2-(1,4-Benzodioxan-2-yl)-äthyl]-4-piperidyl]-2-imidazolidinon ist, seine d- und l-Antipoden und ihre therapeutisch verwendbaren Säureadditionssalze.

7. Pharmazeutische Präparate enthaltend eine Verbindung gemäß einem der Ansprüche 1 bis 6.

8. Die Verbindungen der Ansprüche 1 bis 6 zur Verwendung als Pharmazeutika.

9. Verfahren zur Herstellung von neuen N-Oxacyclyl-alkyl-piperidyl-diazaverbindungen der im Anspruch 1 gezeigten allgemeinen Formel I, worin alle Symbole die im Anspruch 1 angegebenen Bedeutungen haben, und ihren Säureadditionssalzen, dadurch gekennzeichnet, daß man

a) einen reaktionsfähigen Ester eines oxacyclischen Alkanols der allgemeinen Formel III mit einer 1-unsubstituierten Piperidyl-diazaverbindung der allgemeinen Formel IV kondensiert

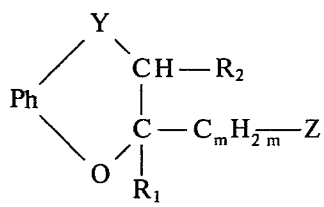

+

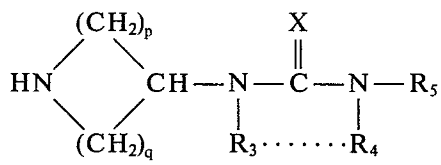

(III) (IV)

worin Z eine reaktionsfähige veresterte Hydroxygruppe bedeutet oder

b) eine Verbindung der allgemeinen Formel V mit dem Kohlensäurederivat der allgemeinen Formel VI umsetzt

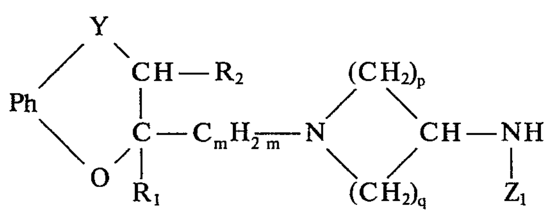

+ $Z_2CX'$

(V) (VI)

worin $Z_1$ für $R_3$ steht oder die Gruppierung $R_3 \ldots R_4-NH-R_5$ bedeutet und $Z_2CX'$ für Ammonium-cyanat oder -thiocyanat, ein Metall-cyanat oder -thiocyanat, einen Niederalkyl-isoharnstoff oder -isothioharnstoff, ein Halogencyan oder Cyanamid, Schwefelkohlenstoff oder Carbonylsulfid, ein Kohlensäurehalogenid oder 1,1-Carbonyldiimidazol bedeutet, mit der Maßgabe, daß mindestens eine der Gruppen $Z_1$ und $Z_2$ Stickstoff enthält oder

c) eine Verbindung der allgemeinen Formel VII

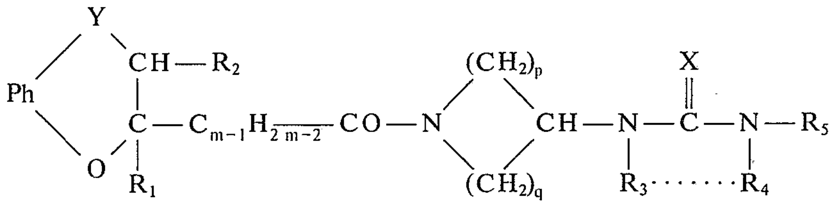

(VII)

reduziert oder

d) eine Verbindung der allgemeinen Formel VIII

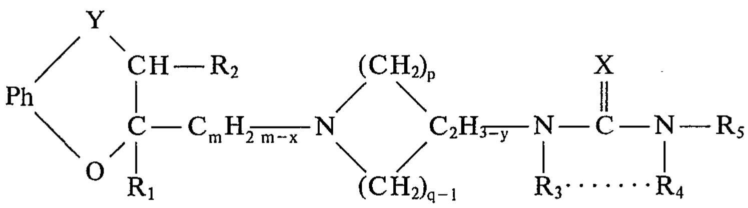

(VIII)

worin jedes der Symbole x und y die Zahl 0 oder 2 bedeutet und ihre Summe x + y für 2 oder 4 steht, hydriert oder

e) eine Verbindung der allgemeinen Formel IX

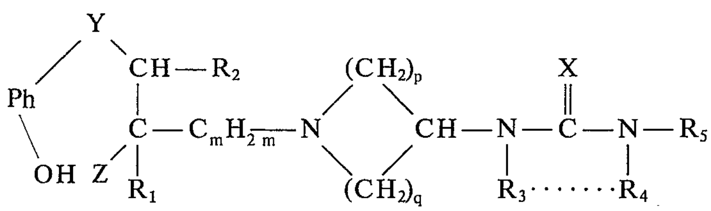

(IX)

worin Z eine reaktionsfähige veresterte Hydroxygruppe bedeutet, ringschließt, und, wenn erwünscht, eine erhaltene Verbindung der Formel I, in welcher Y ein Schwefelatom bedeutet, mit milden Oxydationsmitteln zu ihrem 4-Oxyd oxydiert, und/oder, wenn erwünscht, eine erhaltene freie Verbindung in ein Säureadditionssalz oder ein Säureadditionssalz in die freie Verbindung oder in ein anderes Säureadditionssalz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

## Claims

1. A N-oxacyclylalkylpiperidyldiaza compound of the general formula I

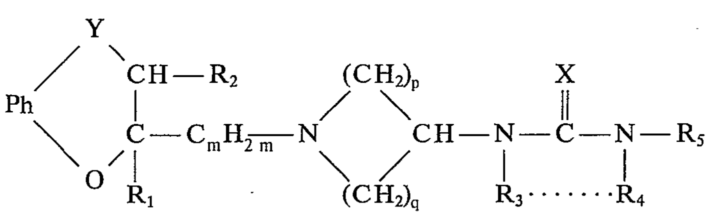

(I)

wherein Ph is unsubstituted 1,2-phenylene or 1,2-phenylene substituted by one to three identical or different members selected from lower alkyl, lower alkoxy, lower alkylenedioxy, halogen or trifluoromethyl, each of $R_1$ and $R_2$ is hydrogen or lower alkyl, each of $R_3$ and $R_4$ is hydrogen, lower alkyl or ($R_3$ + $R_4$) is Ph or lower alkylene separating both nitrogens by 2 to 4 carbon atoms, $R_5$ is hydrogen, lower alkyl or HPh, X is oxo, thioxo, imino or lower alkylimino, Y is epoxy, epithio or sulfinyl, m is an integer from 1 to 7, each of p und q is an integer from 1 to 3, but $(p+q) = 4$, or an acid addition salt thereof.

2. A compound according to claim 1 of the formula I, wherein Ph is 1,2-phenylene, unsubstituted or monosubstituted by lower alkyl, lower alkoxy, lower alkylenedioxy, halogen or trifluormethyl, each of $R_1$ and $R_2$ is hydrogen or lower alkyl, each of $R_3$ and $R_4$ is hydrogen, lower alkyl or ($R_3$ + $R_4$) is Ph or lower alkylene separating both nitrogens by 2 or 3 carbon atoms, $R_5$ is hydrogen, lower alkyl or HPh, X

ist oxo, thioxo, imino or lower alkylimino, Y is epoxy, epithio or sulfinyl, m is an integer from 1 to 4, each of p and q is an integer from 1 to 3, but $(p + q) = 4$, or a therapeutically acceptable acid addition salt thereof.

3. A compound according to claim 1 of the formula I, wherein Ph is 1,2-phenylene unsubstituted or monosubstituted by alkyl or alkoxy, each containing at most 4 carbon atoms, halogen or trifluoromethyl, each of $R_1$, $R_2$ and $R_5$ is hydrogen or alkyl of 4 carbon atoms, each of $R_3$ and $R_4$ is hydrogen or $(R_3 + R_4)$ is alkylene of 2 to 4 carbon atoms separating both nitrogens by 2 or 3 carbon atoms, X is oxo, thioxo or imino, Y is epoxy or epithio, m is an integer from 1 to 4, and each of p und q is 2, or a therapeutically acceptable acid addition salt thereof.

4. A compound according to claim 1 of the general formula II

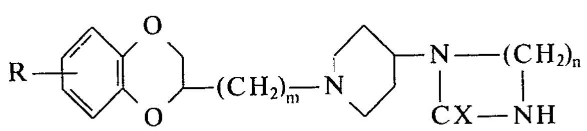

(II)

wherein R is hydrogen, alkyl or alkoxy, each containing at most 4 carbon atoms, halogen or trifluoromethyl, m ist an integer from 1 to 4, n is 2 or 3 and X is oxo, thioxo or imino, or a therapeutically acceptable acid addition salt thereof.

5. 1-[1-[2-(1,4-Benzoxathian-2-yl)-ethyl]-4-piperidyl]-2-imidazolidinone according to claim 1, or a therapeutically acceptable acid addition salt thereof.

6. 1-[1-[2-(1,4-Benzodioxan-2-yl)-ethyl]-4-piperidyl]-2-imidazolidinone according to claim 4, the d- and l-antipodes or a therapeutically accetable acid addition salt thereof.

7. A pharmaceutical preparation containing a compound according to any one of claims 1 to 6.

8. A compound according to any one of claims 1 to 6 for use as a pharmaceutical preparation.

9. A process for the manufacture of a N-oxacyclylalkylpiperidyldiaza compound of the formula I as indicated in claim 1, in which formula all the symbols are as defined in claim 1, or of an acid addition salt thereof, which process comprises

a) condensing a reactive ester of an oxacyclic alkanol of the general formula III with a 1-unsubstituted piperidyldiaza compound of the general formula IV

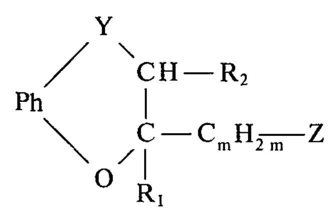

\+

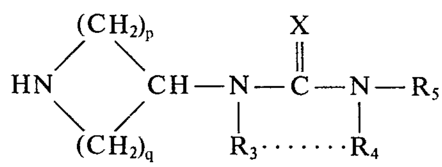

(III) (IV)

wherein Z is a reactive esterified hydroxyl group or

b) reacting a compound of the general formula V with the carbonic acid derivative of the general formula VI

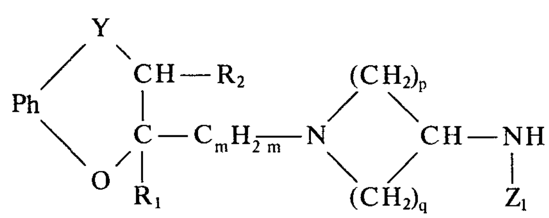

\+ $Z_2CX'$

(V) (VI)

wherein $Z_1$ is $R_3$ or the grouping $R_3 \ldots R_4-NH-R_5$ and $Z_2CX'$ is an ammonium or metal cyanate or thiocyanate, a lower alkyl isourea or isothiourea, a cyanogen halide or amide, carbon disulfide or carbonyl sulfide, a carbonic acid halide or 1,1-carbonyldiimidazole, with the proviso that at least one $Z_1$ and $Z_2$ contains nitrogen, or

c) reducing a compound of the general formula VII

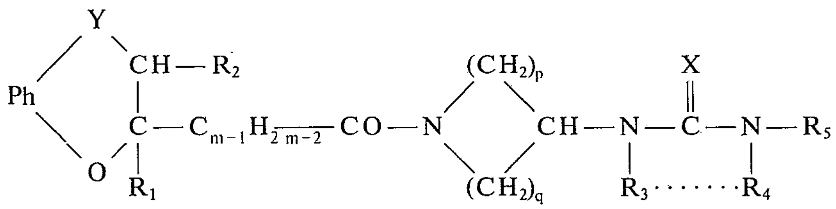

(VII)

or

d) hydrogenating a compound of the general formula VIII

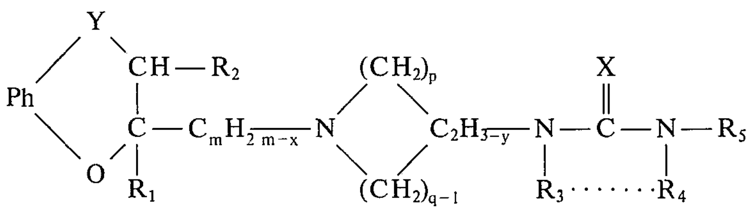

(VIII)

wherein each of x and y is the integer 0 or 2 and the sum of x + y is 2 or 4, or

e) cyclising a compound of the general formula IX

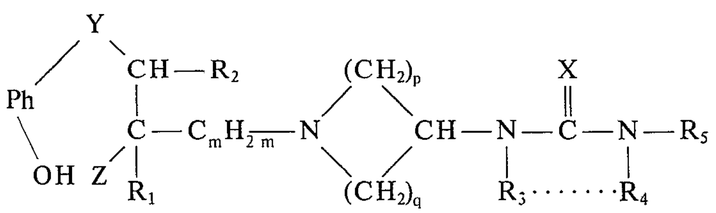

(IX)

wherein Z is a reactive esterified hydroxyl group, and, if desired, converting a resultant compound of formula I, in which Y is a sulfur atom, with mild oxidising agents, into its 4-oxide, and/or, if desired, converting a resultant free compound into an acid addition salt or a resultant acid addition salt into the free compound or into another acid addition salt, and/or, if desired, resolving a mixture of isomers or racemates obtained into the single isomers or racemates, and/or if desired, resolving a racemate obtained into the optical antipodes.

**Revendications**

1. Composés en N-oxacyclyl-alkyl-pipéridyl-diaza, de formule générale I

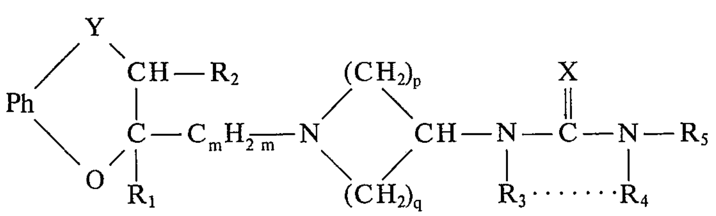

(I)

dans laquelle Ph représente un groupe 1,2-phénylène non substitué ou un groupe 1,2-phénylène portant 1 à 3 substituants identiques ou différents pris dans la classe consistant en les groupes alkyles inférieurs, alcoxy inférieurs, alkylène-dioxy inférieurs, les atomes d'halogènes ou le groupe trifluorométhyle, chacun des symboles $R_1$ et $R_2$ représente l'hydrogène ou un groupe alkyle inférieur, chacun des symboles $R_3$ et $R_4$ représente l'hydrogène ou un groupe alkyle inférieur ou bien ($R_3$ + $R_4$) représente Ph ou un groupe alkylène inférieur qui sépare les deux atomes d'azote par 2 à 4 atomes de carbone, $R_5$ représente l'hydrogène, un groupe alkyle inférieur ou HPh, X représente un groupe oxo, thioxo, imino ou alkylimino inférieur, Y représente un groupe époxy, épithio ou sulfinyle, m est un nombre entier de 1 à 7, chacun des symboles p et q représente un nombre entier de 1 à 3, (p + q) est égal à 4, et leurs sels formés par addition avec des acides.

2. Composés selon la revendication 1, de formule I, dans laquelle Ph représente un groupe 1,2-phénylène non substitué ou monosubstitué par un groupe alkyle inférieur, alcoxy inférieur, alkylène-dioxy inférieur, un halogène ou le groupe trifluorométhyle, chacun des symboles $R_1$ et $R_2$

représente l'hydrogène ou un groupe alkyle inférieur, chacun des symboles $R_3$ et $R_4$ représente l'hydrogène ou un groupe alkyle inférieur, ou bien ($R_3 + R_4$) représente le reste Ph ou un groupe alkylène inférieur qui sépare les deux atomes d'azote par 2 ou 3 atomes de carbone, le symbole $R_5$ représente l'hydrogène, un groupe alkyle inférieur ou HPh, X représente un groupe oxo, thioxo, imino ou alkylimino inférieur, Y représente un groupe époxy, épithio ou sulfinyle, m est un nombre entier de 1 à 4, chacun des symboles p et q représente un nombre entier de 1 à 3, (p + q) est égal à 4, et leurs sels formés par addition avec des acides acceptables pour l'usage pharmaceutique.

3. Composés selon la revendication 1, de formule I, dans laquelle Ph représente un groupe 1,2-phénylène non substitué ou monosubstitué par un groupe alkyle ou alcoxy contenant chacun 4 atomes de carbone au maximum, un halogène ou le groupe trifluorométhyle, chacun des symboles $R_1$, $R_2$ et $R_5$ représente l'hydrogène ou un groupe alkyle à 4 atomes de carbone, chacun des symboles $R_3$ et $R_4$ repésente l'hydrogène ou bien ($R_3 + R_4$) représente un groupe alkylène en C 2-C 4 qui sépare les deux atomes d'azote par 2 ou 3 atomes de carbone, X représente un groupe oxo, thioxo ou imino, Y représente un groupe épithio, m est un nombre entier de 1 à 4, chacun des symbo es p et q représente le nombre 2, et leurs sels formés par addition avec des acides utilisables en thérapeutique.

4. Composés selon la revendication 1, répondant à la formule générale II

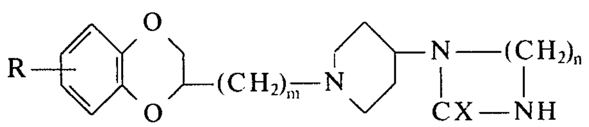

dans laquelle R représente l'hydrogène, un groupe alkyle ou alcoxy contenant chacun 4 atomes de carbone au maximum, un halogène ou le groupe trifluorométhyle, m est un nombre entier de 1à 4, n est un nombre entier égal à 2 ou 3, et X représente un groupe oxo, thioxo ou imino, et leurs sels formés par addition avec des acides utilisables en thérapeutique.

5. Un composé selon la revendication 1, qui est la 1-[1-[2-(1,4-benzoxathianne-2-yl)-éthyl]-4-pipéridyl-2-imidazolidinone et ses sels formés par addition avec des acides utilisables en thérapeutique.

6. Un composé selon la revendication 4, qui est la 1-[1-[2-(1,4-benzodioxanne-2-yl)-éthyl]-4-pipéridyl]-2-imidazolidinone, ses antipodes d et l et leurs sels formés par addition des acides utilisables en thérapeutique.

7. Préparations pharmaceutiques contenant un composé selon l'une des revendications 1 à 6.

8. Les composés des revendications 1 à 6, pour l'utilisation en tant que produits pharmaceutiques.

9. Procédé de préparation des nouveaux composés en N-oxacyclyl-alkyl-pipéridyl-diaza de formule générale I de la revendication 1, et de leurs sels formés par addition avec des acides, caractérisé en ce que:

a) on condense un ester réactif d'un alcanol oxacyclique de formule générale III avec un composé en pipéridyl-diaza non substitué en position 1, de formule générale IV

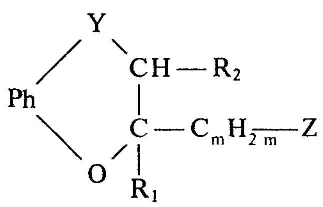

+

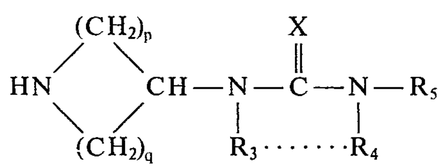

(III) (IV)

Z représentant un groupe hydroxy estérifié réactif, ou bien

b) on fait réagir un composé de formule générale V avec le dérivé d'acide carbonique de formule générale VI

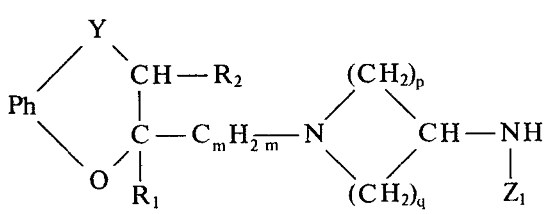

+ $Z_2CX'$

(V) (VI)

$Z_1$ représentent $R_3$ ou bien le groupement $R_3 \ldots R_4 - NH - R_5$ et $Z_2CX'$ le cyanate d'ammonium ou le thiocyanate d'ammonium, un cyanate ou thiocyanate métallique, une (alkyle inférieur)-isourée ou -isothiourée, un cyanure d'halogène ou le cyanamide, le sulfure de carbone ou le sulfure de

carbonyle, un halogénure de l'acide carbonique ou le 1,1-carbonyldiimidazole, étant spécifié que l'un au moins des groupes $Z_1$ et $Z_2$ contient de l'azote, ou bien

c) On réduit un composé de formule VII

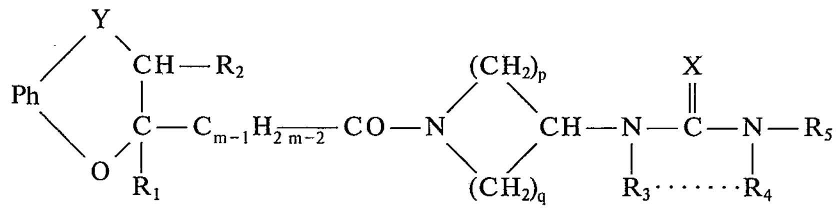

(VII)

ou bien

d) on hydrogène un composé de formule générale VIII

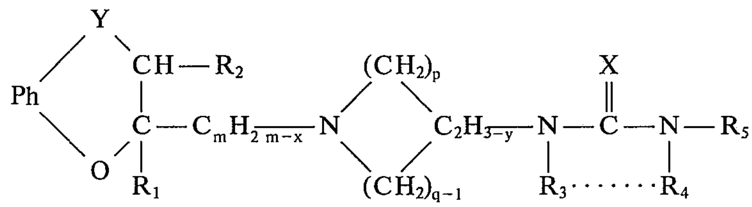

(VIII)

dans laquelle chacun des symboles x et y représente 0 ou 2, et la somme x + y est égale à 2 ou 4, ou bien

e) on cyclise un composé de formule générale IX

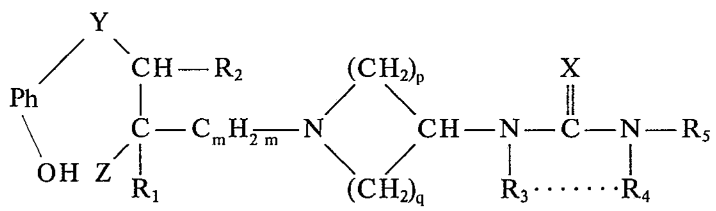

(IX)

dans laquelle Z représente un groupe hydroxy estérifié réactif et, si on le désire, ayant obtenu un composé de formule I dans laquelle Y représente un atome de soufre, on l'oxyde en son 4-oxyde à l'aide d'agents oxydants ménagés et/ou, si on le désire, ayant obtenu un composé libre, on le convertit en un sel par addition avec un acide ou ayant obtenu un sel formé par addition avec un acide, on le convertit en le composé libre ou en un autre sel formé par addition avec un acide, et/ou, si on le désire, ayant obtenu un mélange d'isomères ou de racémates, on les sépare en les isomères ou racémates individuels, et/ou, si on le désire, ayant obtenu des racémates, on les résoud en les antipodes optiques.